# EUROPEAN PATENT SPECIFICATION

(11) **EP 2 309 263 B1**
(45) Date of publication and mention of the grant of the patent: **17.06.2015**
(21) Application number: 09075425.0
(22) Date of filing: 14.09.2009
(51) Int. Cl.: C07K 16/00, C07K 14/705

(54) **Chimeric IgY Fc receptor constructs**
Chimerische IgYFc-Rezeptorkonstrukte
Constructions de récepteur IgY Fc chimères

(43) Date of publication of application: 13.04.2011
(73) Proprietor: PLS-Design GmbH, 20255 Hamburg (DE)
(72) Inventor: Braren, Ingke, 22303 Hamburg (DE); Grunwald, Thomas, 22459 Hamburg (DE)
(74) Representative: Jungblut, Bernhard Jakob

(56) References cited:
- EP-A- 1 777 524
- VIERTLBOECK B.C. ET AL.: "The chicken leukocyte receptor complex encodes a primordial, activating, high-affinity IgY Fc receptor" PROC. NAT. ACAD. SCI., vol. 104, no. 28, 10 July 2007 (2007-07-10), pages 11718-11723, XP002559219
- BOSCATO L M ET AL: "HETEROPHILIC ANTIBODIES: A PROBLEM FOR ALL IMMUNOASSAYS" CLINICAL CHEMISTRY, AMERICAN ASSOCIATION FOR CLINICAL CHEMISTRY, WASHINGTON, DC, vol. 34, no. 1, 1 January 1988 (1988-01-01), pages 27-33, XP002918652 ISSN: 0009-9147
- LARSSON A ET AL: "Chicken antibodies: a tool to avoid interference by complement activation in ELISA" JOURNAL OF IMMUNOLOGICAL METHODS, ELSEVIER SCIENCE PUBLISHERS B.V.,AMSTERDAM, NL, vol. 156, no. 1, 25 November 1992 (1992-11-25), pages 79-83, XP023986711 ISSN: 0022-1759 [retrieved on 1992-11-25]
- LARSSON A ET AL: "USE OF CHICKEN ANTIBODIES IN ENZYME IMMUNOASSAYS TO AVOID INTERFERENCE BY RHEUMATOID FACTORS" CLINICAL CHEMISTRY, AMERICAN ASSOCIATION FOR CLINICAL CHEMISTRY, WASHINGTON, DC, vol. 37, no. 3, 1 January 1991 (1991-01-01), pages 411-414, XP002434637 ISSN: 0009-9147
- GREUNKE K ET AL: "Bivalent monoclonal IgY antibody formats by conversion of recombinant antibody fragments" JOURNAL OF BIOTECHNOLOGY, ELSEVIER SCIENCE PUBLISHERS, AMSTERDAM, NL, vol. 124, no. 2, 13 July 2006 (2006-07-13), pages 446-456, XP024956743 ISSN: 0168-1656 [retrieved on 2006-07-13]

## Description

### Field of the invention

The invention relates to chimeric constructs comprising molecules from different organisms, nucleic acids coding therefore, an expression vector comprising such nucleic acids, methods of producing such constructs and uses thereof.

### State of the art and background of the invention

Chicken IgY, the equivalent of mammalian IgG, is the major low molecular weight serum immunoglobulin in oviparous (egg laying) animals. It is called IgY rather than IgG to distinguish it from its mammalian counterpart (Leslie GA, Clem LW, J Exp Med 130, 1337-1352, 1969). The overall structure of IgY is similar to mammalian IgG, with two light and two heavy chains. The light chain is lighter and the heavy chain of the IgY molecule is larger than its mammalian counterpart. The molecular mass has been reported to be approx. 167 kDa, slightly larger than IgG (approx. 160 kDa) (Sun S, et al., Rapid Commun Mass Spectrom 15, 708-712, 2001). The H chain ( approx. 65 kDa), called upsilon (U, capital letter Y) has one variable (V) region and four constant (C) regions. The light chain (approx 18 kDa) is composed of one variable and one constant domain.

The phylogenetic difference between avian and mammalian species provides access to a different antibody repertoire than the traditional mammalian antibodies. IgY antibodies recognize other epitopes than mammalian antibodies. Furthermore, IgY antibodies raised against mammalian proteins in chicken are known to bind to more epitopes than corresponding mammalian antibodies. For example, it has been shown that 3-5 times more chicken antibody than swine antibody will bind to rabbit IgG which will amplify the signal in an immunological assay (Horton J, et al., J Invest Dermatol 85, 96-99, 1984). In addition, for the production of antibodies against conserved mammalian proteins chicken is a better choice than e.g. rabbits, since the immune response usually increases as the difference between the antigen and the immunized animal increases (Horton J, et al., J Invest Dermatol 85, 96-99, 1984).

Due to the phylogenetic difference between avian IgY and mammalian IgG, IgY molecules provide several important advantages for diagnostic applications. One important advantage is the lack of cross-reactivity with mammalian antibodies. Utilizing mammalian antibodies as secondary anti-mammalian Immunoglobulin antibodies in immunoassays, these antibodies may cross-react with mammalian capture antibodies in ELISA-type assays or in histological analyses with bovine IgG in the bovine serum albumin solution often used for blocking purposes. The prevalence of human anti-mammalian antibodies (heterophilic antibodies) causing potential interferences in immunological assays, varies from 1-80 % in the general population (Kricka LJ, Clin. Chem. 45, 942-956, 1999). The rheumatoid factor (RF) and human anti-mouse IgG antibodies (HAMA) are probably the most well known causes of false positive or false negative reactions in immunological assays (Boscato LM, Stuart MC, Clin Chem 34, 27-33, 1988). RF is an auto-antibody that reacts with the Fc part of mammalian IgG. The disease most often associated with RF is rheumatoid arthritis, but RF can be found in serum from patients with many other diseases and also in 3-5% of healthy blood donors (Johnson PM, Faulk WP, Clin Immunol Immunopathol 6, 414-430, 1976). Production of HAMA is mainly the result of therapeutic approaches with mouse monoclonal antibodies, but HAMA may also be found in serum from patients who have not been treated with antibodies. RF or HAMA may react with both the capture antibody and the detection antibody in a sandwich assay, thereby mimicking antigen activity. A reaction with the detection antibody results in formation of an immune complex which may influence the activity of the detection antibody. HAMA may also react with the antigen-binding region of the detection antibody, thereby impairing or inhibiting antigen binding. The problem of RF and HAMA interference will increase as the sensitivity of the assay increases. Interference by anti-IgG antibodies and antibody-binding substances have been demonstrated in approximately 40% of serum samples from healthy individuals in an immunoradiometric assay (Boscato L, Stuart M, Clin Chem 32, 1491-1495, 1986). RF and HAMA will also give erroneous results in nephelometry and turbidimetry as they change the size of antigen-antibody complex (Chambers RE, et al., Ann Clin Biochem 24, 520-524, 1987). Since heterophilic antibodies including RF or HAMA do not react with chicken IgY antibodies, avian IgY can be used to avoid interference due to these factors (Larsson A, et al., Clin Chem 37, 411-414, 1991; Larsson A, Mellstedt H,. Hybridoma 11, 33-39, 1992).

Another important advantage of the phylogenetic difference between avian and mammalian species is the lack of human complement activation by IgY antibodies. In clinical laboratories, most analyses are performed on serum samples. A newly obtained serum sample contains active complement, but the activity declines during storage and handling (Whaley, K. Methods in complement for clinical immunologists, Churchill Livingstone, 1985). Accordingly, the complement activity may vary between different patients and also between different samples from the same patient. To avoid activation of the complement cascade, EDTA is often included in tubes used for blood sampling. EDTA prevents complement activation and coagulation by sequestering calcium ions. Most of the standards and controls used have been stored and contain an inactive complement system. This difference in activity between the samples and the standards will cause erroneous results. Mammalian antibodies bound to a solid phase and antigen-antibody complexes containing mammalian antibodies can activate the human complement system (Larsson A, Sjoquist J, J Immunol Methods 119, 103-109, 1989). Activated C4 molecules bind to the Fab region of IgG and may interfere with the antigen binding (Campbell RD, et al., Biochem J 189, 6780, 1980). Complement components may also solubilize precipitated immune complexes and prevent soluble immune complexes from precipitating (Baatrup G, et al., Scand J Immunol 23, 397-406, 1986; .Miller GW, Nussenzweig V, Proc Natl Acad Sci USA 72, 418-422, 1975). Such complement activation was shown to interfere in an immunometric TSH (thyroidea stimulating hormone) assay and depressed the TSH values by up to 40 % (Kapyaho K, et al., Scand J Clin Lab Invest 49, 211215, 1989). Because chicken antibodies do not activate the human complement system, they can be used as to reduce interference by complement activation (Larsson A, et al., J Immunol Methods 156, 79-83, 1992).

In addition to the above mentioned advantages, IgY antibodies do not interact with human Fc receptors which are found on many types of blood cells (van de Winkel JG, Capel PJ, Immunol Today 14, 215-221, 1993). Human Fc(RI has a high affinity for monomeric mammalian IgG, while Fc(RII and Fc(RIII mainly bind mammalian IgG complexes. There is often some aggregated IgG formed during the purification of IgG or during the labeling procedures that will increase the binding to Fc(RII and Fc(RIII receptors. Interaction with Fc receptors may cause an increased background staining in immunological tissue analysis. When working with living cells, the interaction with Fc receptors may cause cell activation and changes in the expression of surface proteins. For example, it has been shown that mammalian antibodies used in flow cytometry form immune complexes that cause platelet activation and changes in the expression of the GpIlb-IIIa receptor. No activation was observed when chicken antibodies were used (Lindahl TL, et al., Thromb Haemost 68, 221-225, 1992).

Furthermore, the use of IgY eliminates also unwanted bacterial Fc-receptor interactions. Staphylococcal protein A and Streptococcal protein G are Fc-binding bacterial proteins which are widely used for their ability to bind to mammalian IgG. Bacteria of the *Staphylococcus aureus* Cowan 1 strain and group C Streptococcus sp. are used as immunoadsorbent for mammalian IgG. Staphylococci and Streptococci are often found in bacterial samples. When present, they may bind detection antibodies with specificities for other bacteria and cause erroneous results. Chicken antibodies do not react with protein A or protein G and can be used to reduce interference problems due to bacterial Fc receptors (Hoffman WL, et al., J Immunol Methods 198, 67-77, 1996).

In summary, IgY antibodies offer great opportunities for diagnostic applications. However, there are also problems associated with the IgY technology. In contrast to immunoassays based on mammalian antibody technology, the availability of reagents for the detection of avian IgY antibodies is limited. Furthermore, most of the few available monoclonal anti-IgY antibodies are not sufficiently characterized. Polyclonal anti-IgY antibodies offer an inexpensive alternative, but they suffer from the known disadvantages of polyclonal antibodies including varying specificities and affinities of different antibody batches.

An attractive alternative to monoclonal anti-IgY antibodies represents a high-affinity IgY Fc receptor of avian origin, designated CHIR-AB1, which has been identified and cloned recently (Viertlboeck BC, et al., Proc. Natl. Acad. Sci, USA 104: 11718-11723, 2007). The receptor is a member of the chicken Ig-like receptor (CHIR) multigene family localized in the leukocyte receptor complex (LRC) and consists of single extracellular Ig domain, a basic transmembrane charge, and a cytoplasmic immunoreceptor tyrosine-based inhibitory motif (ITIM). The CHIR-AB1 ectodomain (residues 1-93 of the mature protein) has been expressed and secreted in insect cells, crystallized, and structurally analyzed at a 1.8 Å resolution (Arnon, TI, et al., J Mol Biol381: 1012-1024, 2008). A fusion protein with the single CHIR-AB1 Ig domain fused to the hinge region, CH2 and CH3 domain of human IgG1 was generated to test its affinity and specificity by surface plasmon resonance and by ELISA analyses, respectively (Viertlboeck BC, et al., Proc. Natl. Acad. Sci, USA 104: 11718-11723, 2007). The data demonstrate that CHIR-AB1 is a high-affinity receptor that specifically binds FcY with an equilibrium constant (K_{D}) of 5.4 nM and IgY with a K_{D} of 27 nM. A potential application of the solubilized receptor construct as detection reagent of avian IgY antibodies in immunoassays is not mentioned.

By comparison of CHIR-AB1-like sequences in databases, 18 homologues of CHIR-AB1 were identified and cloned (Viertlboeck BC, et al., J Immunol 182: 6985-6992, 2009a). After expression of the extracytoplasmic Ig domain of the homologues as fusion proteins with the hinge region, CH2 and CH3 domain of human IgG1, analyses revealed that six of these fusion proteins recognized IgY with an equilibrium constant between 25 nM for high binders and 260 nM for low binders. A potential application of these receptor constructs as detection reagent of avian IgY antibodies in immunoassays is not mentioned.

An additional FcR-related gene in the chicken, designated gallus gallus FcR (ggFcR), has been identified very recently (Viertlboeck BC, et al., J Immunol 182: 1533-1540, 2009b). The receptor consisting of four extracellular C2-set Ig domains, a transmembrane region containing arginine as a positively charged amino acid, and a short cytoplasmic tail, binds selectively chicken IgY. Surprisingly, ggFcR is highly related to chicken LCR-encoded genes, but is located on a chromosomal region distinct from the LCR and FcR gene clusters. A potential application of this receptor as detection reagent of avian IgY antibodies in immunoassays is not mentioned.

While extracellular Ig domains of high-affinity IgY Fc receptor of avian origin, encoded either by LCR-genes, FcR genes or by genes located on other chromosomal regions, provide suitable reagents for the detection of avian IgY antibodies in immunoassays, they are not applicable as substitute of mammalian antibodies in clinically established immunoassays based on reagents of mammalian origin. For example, detection of antigens in a sandwich ELISA requires a detection step with a secondary antibody derived from a different mammalian species than the capture antibody. To allow for high sensitivity, in many assays bound secondary antibodies are visualized by labeled anti-secondary antibodies, thereby introducing a signal amplification step. Replacing the secondary mammalian antibodies by avian IgY antibodies in clinically established immunoassays, however, the last step would require a costly development of novel reagents. Therefore, there is a need in the field to develop a technology that allows cost-effective substitution of mammalian antibodies by IgY in clinically established immunoassays.

Based on their advantageous properties, IgY antibodies represent also excellent tools for standardization of immunoassays. For the vast majority of immunoassays, however, application of IgY antibodies as standardization reagents is not feasible since currently available detection reagents of bound IgY antibodies are not compatible with clinically utilized detection reagents. For example, detection of allergen-specific IgE antibodies in human sera by ELISA includes binding of serum IgE to immobilized allergens followed by a detection step with anti-human IgE antibodies. Utilizing allergen-specific IgY antibodies for standardization of such assays, anti-human IgE antibodies cannot be applied. Since results obtained with anti-IgY antibodies are not comparable to those obtained with anti-human IgE antibodies, there is a need in the field to develop reagents for the detection of IgY antibodies which can be adapted to detection procedures for mammalian immunoglobulins.

Similar considerations apply to the use of IgY antibodies as substitutes of antigen- or allergen-specific immunoglobulins in human sera. Since manufacturers of immunoassays have to provide a positive control serum for each assay, artificial sera generated by the addition of variable amounts of antigen- or allergen-specific IgY to sera from healthy individuals could solve urgent problems in fields where only few sera and very limited serum quantities are available. For example, sera from young children suffering from milk allergy are difficult to obtain and serum quantities are extremely limited. Likewise, manufacturers of immunoassays designed for the diagnosis of rare diseases are struggling to provide sufficient positive control sera. Therefore, IgY-containing artificial sera offer an attractive solution as positive control sera for such assays. The acceptance of artificial sera based on IgY antibodies, however, depends on reagents for the detection of IgY antibodies which can be adapted to detection procedures for mammalian immunoglobulins. Since such versatile reagents are not available, there is an urgent need in the field.

A fusion protein comprising a human binding moiety for human IgE an IgY constant immunoglobulin domains of avian origin is known from EP 1777524 A.

### Technical Problem of the invention

A first object of the present invention is to provide reagents for the detection of IgY antibodies which can be adapted to detection procedures for mammalian immunoglobulins.

A second object of the present invention is to provide a high-affinity reagent for the detection of antigen- or allergen-specific IgY antibodies which can be adapted to detection procedures for different immunoglobulins.

### Summary of the invention an preferred embodiments

In order to solve the above-mentioned objects the invention provides the subject matters of the claims. These are described in the following in more detail. With respect to explanations and definitions provided, these generally apply to all claims without explicit reference thereto.

In one embodiment, the present invention provides a chimeric fusion construct or protein comprising the extracellular portion of an avian high-affinity IgY Fc receptor or the extracellular portion of an avian FcR-related molecule with a high-affinity IgY binding capability, and at least one mammalian constant immunoglobulin domain. The format of these constructs can be monomeric or homodimeric. The chimeric constructs combine the IgY binding characteristics of an avian high-affinity IgY Fc receptor with mammalian constant immunoglobulin which can be detected by anti-mammalian antibodies or other reagents capable of specifically binding to mammalian constant immunoglobulin domains. The chimeric fusion constructs are useful for the application of avian IgY antibodies as substitutes of mammalian antibodies for diagnostic purposes, in that the chimeric fusion construct of the invention allows to detect and quantify IgY antibodies by utilizing detection procedures developed for mammalian immunoglobulins.

In a variant of this embodiment at least two mammalian constant immunoglobulin domains, each derived from a different antibody isotype of the same mammalian species, are comprised. Such chimeric fusion constructs are useful for the application of avian IgY antibodies as substitutes of mammalian antibodies for diagnostic purposes, in that the chimeric fusion construct of the invention allows to detect and quantify IgY antibodies by utilizing detection procedures developed for different immunoglobulins of the same mammalian species. Thereby, one chimeric fusion construct can be used for the detection of different antibody isotypes, depending on the number of constant domains from different antibody isotypes in the chimeric fusion construct. The at least two mammalian constant immunoglobulin domains may each be derived from the same or different mammalian species.

The present invention further provides nucleic acid molecules encoding a fusion protein of the invention. In a specific embodiment, the nucleic acid molecules of the invention encode a chimeric fusion construct comprising the extracellular portion of the avian leukocyte receptor CHIR-AB1 (CHIR-AB1-ecd) and at least one mammalian constant immunoglobulin domain, or at least two constant immunoglobulin domains each derived from different antibody isotypes of the same mammalian species, or at least two constant immunoglobulin domains each derived from different mammalian species.

In a further specific embodiment, the present invention provides a chimeric fusion construct or protein comprising the extracellular portion of the avian leukocyte receptor CHIR-AB1 (CHIR-AB1-ecd) and at least one mammalian constant immunoglobulin domain, or at least two constant immunoglobulin domains each derived from different antibody isotypes of the same mammalian species, or at least two constant immunoglobulin domains each derived from different mammalian species.

The present invention further provides expression vectors comprising the nucleic acids of the invention operationally associated with a promoter. The present invention also provides methods for producing the polypeptides encoded by the nucleic acids of the invention. In particular, the present invention provides for culturing mammalian and prokaryotic cells transformed with an expression vector of the invention so that the chimeric fusion proteins of the invention are expressed by the cells, and recovering these polypeptides so expressed from the culture.

In a specific embodiment of the invention, the chimeric fusion proteins of the invention are applied as reagents for the detection of secondary antigen- or allergen-specific IgY antibodies in immunological assays originally established for mammalian detection antibodies. In another specific embodiment of the invention, the chimeric fusion proteins of the invention with at least two constant immunoglobulin domains each derived from different antibody isotypes of the same mammalian species, are applied as reagents for the detection of secondary antigen- or allergen-specific IgY antibodies in immunological assays originally established for different detection antibody isotypes of the same mammalian species. In still another specific embodiment of the invention, the chimeric fusion proteins of the invention with at least two constant immunoglobulin domains each derived from different mammalian species, are applied as reagents for the detection of secondary antigen- or allergen-specific IgY antibodies in immunological assays originally established for mammalian detection antibodies of different mammalian species.

In another specific embodiment of the invention, the chimeric fusion proteins of the invention are applied as reagents for the detection of IgY-based standardization reagents in immunological assays designed for the determination of mammalian antibodies. In another specific embodiment of the invention, the chimeric fusion proteins of the invention with at least two constant immunoglobulin domains, each derived from different antibody isotypes of the same mammalian species, are applied as reagents for the detection of IgY-based standardization reagents in immunological assays designed for the determination of different mammalian antibody isotypes of the same mammalian species. In still another specific embodiment of the invention, the chimeric fusion proteins of the invention with at least two constant immunoglobulin domains each derived from different mammalian species, are applied as reagents for the detection of IgY-based standardization reagents in immunological assays designed for the determination of mammalian d antibodies from different mammalian species.

In another specific embodiment of the invention, the chimeric fusion proteins of the invention are used in combination with antigen- or allergen-specific IgY antibodies to generate artificial mammalian sera for application as positive control sera in immunoassys designed for the determination of mammalian antibodies. In another specific embodiment of the invention, the chimeric fusion proteins of the invention with at least two constant immunoglobulin domains each derived from different antibody isotypes of the same mammalian species, are used in combination with antigen-or allergen-specific IgY antibodies to generate artificial mammalian sera for application as positive control sera in immunoassys designed for the determination of different mammalian antibody isotypes of the same mammalian species. In still another specific embodiment of the invention, the chimeric fusion proteins of the invention with at least two constant immunoglobulin domains each derived from different mammalian species, are used in combination with antigen- or allergen-specific IgY antibodies to generate artificial mammalian sera for application as positive control sera in immunoassays designed for the determination of mammalian antibodies from different mammalian species.

Also provided by the invention is a kit that includes, in one or more containers or packages, antigen- or allergen-specific IgY antibodies, a fusion protein of the invention, appropriate buffer solutions, washing solutions and suitable reagents for the detection of the fusion protein of the invention.

### Definitions

Unless otherwise defined, all technical and scientific terms used herein have the same meaning as commonly understood by one of ordinary skill in the art to which this invention belongs. Although methods and materials similar or equivalent to those described herein can be used in practicing or testing the present invention, suitable methods and materials are described below. All publications, patent applications, patents, and other references mentioned herein are incorporated by reference in their entirety. In the case of conflict, the present specification, including definitions, will control. In addition, the materials, methods, and examples are illustrative only and not intended to be limiting.

A "nucleic acid molecule" refers to the phosphate ester polymeric form of ribonucleosides (RNA molecules) or deoxy ribonucleosides (DNA molecules) in either single stranded form, or double stranded form. Double strand DNA-DNA, DNA-RNA, and RNA-RNA helices are possible. The term nucleic acid molecule refers to the primary and secondary structure of the molecule, but does not limit it to any particular tertiary forms. Thus, this term includes double stranded DNA found in linear or circular DNA molecules (e.g., restriction fragments), viruses, plasmids, and chromosomes. DNA sequences may be described herein according to the normal convention of giving only the sequence in the 5' to 3' direction along the nontranscribed strand of DNA (i.e., the strand having a sequence homologous to the mRNA). A recombinant DNA molecule is a DNA molecule that has undergone a molecular biological manipulation.

A DNA "coding sequence" is a double-stranded DNA sequence which is transcribed and translated into a polypeptide in vivo when placed under the control of appropriate regulatory sequences. The boundaries of the encoding sequence are determined by a start codon at the 5' (amino) terminus and a translation stop codon at the 3' (carboxyl) terminus. A coding sequence can include, but is not limited to, prokaryotic sequences, cDNA from eukaryotic mRNA, genomic DNA sequences from eukaryotic (e.g., mammalian) DNA, and even synthetic DNA sequences. If the coding sequence is intended for expression in a eukaryotic cell, a polyadenylation signal and transcription termination sequence will usually be located 3' to the coding sequence. Transcriptional and translational control sequences are DNA regulatory sequences, such as promoters, enhancers, terminators, and the like, that provide for the expression of a coding sequence in a host cell. A promoter sequence is a DNA regulatory region capable of binding RNA polymerase in a cell and initiating transcription of a downstream (3' direction) coding sequence. For purposes of the present invention, the promoter sequence is bounded at its 3' terminus by the transcription initiation site and extends upstream (5' direction) to include the minimum number of bases or elements to initiate transcription at levels detectable above background. Within the promoter sequence will be found a transcription initiation site, as well as protein binding domains (consensus sequences) responsible for the binding of RNA polymerase. Eukaryotic promoters will often, but not always, contain "TATA" boxes and "CAT" boxes. A coding sequence is under the control of transcriptional and translational control sequences in a cell when RNA polymerase transcribes the coding sequence into mRNA, which is then translated into the protein encoded by the coding sequence. A signal sequence can be included before the coding sequence. This sequence encodes a signal peptide, N-terminal to the polypeptide, that directs the host cell to transport the polypeptide to the cell surface or secrete the polypeptide into the media, and this signal peptide is usually selectively degraded by the cell upon exportation. Signal sequences can be found associated with a variety of proteins native to prokaryotes and eukaryotes. In accordance with the present invention there may be employed conventional molecular biology, microbiology, and recombinant DNA techniques within the skill of the art. Such techniques are explained fully in the literature.

As used herein, the term "coding region" refers to the nucleotide sequences that encode the amino acid sequences found in the nascent polypeptide as a result of translation of an mRNA molecule. The coding region is bounded in eukaryotes, on the 5' side by the nucleotide triplet "ATG" that encodes the initiator methionine and on the 3' side by one of the three triplets which specify stop codons (i.e., TAA, TAG, and TGA). The term "encoding" or "encodes" refers to said "coding sequence" and said "coding region".

As used herein, the term "amino acid sequence" is recited herein to refer to an amino acid sequence of a protein molecule. The term "amino acid sequence" and like terms such as "polypeptide" or "protein" are not meant to limit the amino acid sequence to the complete, native amino acid sequence associated with the recited protein molecule. Rather the terms "amino acid sequence" and "protein" encompass partial sequences, fragments of the protein or polypeptide and modified sequences.

The term "wild type" refers to a nucleic acid molecule, a gene or gene product that has the characteristics of that nucleic acid molecule, gene or gene product when isolated from a naturally occurring source. A wild type gene is that which is most frequently observed in a population and is thus arbitrarily designated the "normal" or "wild-type" form of the gene.

In contrast, the terms "modified," "mutant", and "comprising a mutation" refer to a nucleic acid molecule, gene or gene product that displays modifications in sequence and or functional properties (i.e., altered characteristics) when compared to the wild-type nucleic acid molecule, gene or gene product. In some embodiments, the modification comprises at least one insertion, deletion, or substitution of one or more nucleotides or amino acids, respectively. A "mutation" in a nucleic acid sequence or gene can lead to an amino acid substitution in the corresponding amino acid sequence, wherein the amino acid substitution can be "conservative" or "non-conservative" or "random".

A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophane), beta-branched side chains (e.g., threonine, valine, isoleucine), and aromatic side chains (e.g., tyrosine, phenylalanine, tryptophane, histidine). Thus, a predicted non-essential amino acid residue in a polypeptide according to the invention, fragments, and analogs thereof is preferably replaced with another amino acid residue from the same side chain family.

In the context of the present invention, the term "nucleic acid homology" is equivalent to "nucleic acid identity". In the context of the present invention, the term "amino acid homology" is equivalent to "amino acid identity". The percent homology between two sequences is a function of the number of identical positions shared by the sequences (i.e., percent homology equals the number of identical positions divided by the total number of positions times 100). For comparison purposes the sequences are aligned, wherein gaps can be introduced in the sequence of a first amino acid sequence for optimal alignment with a second amino acid sequence.

In the context of the present invention, the term "amino acid homology" is equivalent to "amino acid identity". The percent homology between two sequences is a function of the number of identical positions shared by the sequences (i.e., percent homology equals the number of identical positions divided by the total number of positions times 100). For comparison purposes the sequences are aligned, wherein gaps can be introduced in the sequence of a first amino acid sequence for optimal alignment with a second amino acid sequence.

The term "recombinant DNA molecule" as used herein refers to a DNA molecule that is comprised of segments of DNA joined together by means of molecular biological techniques. Similarly, the term "recombinant protein" or "recombinant polypeptide" or "recombinant antibody" as used herein refers to a protein molecule that is expressed from a recombinant DNA molecule.

The terms "peptide," "peptide sequence," "amino acid sequence," "polypeptide," and "polypeptide sequence" are used interchangeably herein to refer to at least two amino acids or amino acid analogs, which are covalently linked by a peptide bond or an analog of a peptide bond. The term "peptide" includes oligomers ("oligopeptides") and polymers ("polypeptides") of amino acids or amino acid analogs. The term "peptide" also includes molecules commonly referred to as peptides, which generally contain from about two (2) to about twenty (20) amino acids. The term "peptide" also includes molecules commonly referred to as polypeptides, which generally contain from about twenty (20) to about fifty amino acids (50). The term "peptide" also includes molecules commonly referred to as proteins, which generally contain from about fifty (50) to about three thousand (3000) amino acids. Examples of a "polypeptide" or "protein" are the polypeptides of the invention. The amino acids of the peptide may be L-amino acids or D-amino acids. A peptide, polypeptide or protein may be synthetic, recombinant or naturally occurring. A synthetic peptide is a peptide produced by artificial means *in vitro.*

The term "native protein" as used herein refers to a protein that does not contain amino acid residues encoded by vector sequences. That is the native protein contains only those amino acids found in the protein as it occurs in nature. A native protein may be produced by recombinant means or may be isolated from a naturally occurring source.

The term "fragment" when used in reference to a nucleotide sequence (as in "fragment of a given nucleotide sequence") refers to partial segments of that sequence. The fragments may range in size from 4 nucleotides to the entire nucleotide sequence minus one nucleotide (10 nucleotides, 20, 30, 40, 50, 100, 200, etc.).

Similarly, the term "fragment" when used in reference to a polypeptide sequence refers to partial segments of that sequence. In some embodiments, the fragment has an amino-terminal and/or carboxy-terminal deletion as compared to the native protein, but the remaining amino acid sequence is identical to the corresponding positions in the amino acid sequence deduced from a full-length cDNA sequence.

As used herein, the term "derivative or analog thereof" refers to any nucleic acid related to the nucleic acids of the invention and to any polyeptide related to the polypeptides of the invention. In a derivative or analog according to the invention, the structure of the polypeptide can be modified to increase resistance to proteolytic degradation in vivo. For this purpose, amino acid residues of a polypeptide of the invention can be substituted by D-amino acids, non-natural amino acids or non-amino acid analogs, or such non-natural amino acids and analogs can be added to produce a modified peptide within the scope of the invention.

A "derivative" according to the invention can be selected from the group consisting of a mutant comprising one or more amino acid substitutions, a mutant comprising one or more amino acid substitutions that introduce one or more sensitive sites for site-specific proteases, and a fusion protein comprising a chimeric IgY Fc receptor construct of the invention and a C- or N-terminal fused polypeptide suitable for affinity purification. Polypeptides suitable for affinity purification are selected from a group including, but not limited to, an oligo- or poly-histidine tag (His tag), the maltose binding protein (MBP), a chitin binding domain (CBD), a streptavidin derivative, other antibody epitope-containing polypeptides, and polypeptides suitable for hapten derivatization *in vitro* and/or *in vivo.*

The term "chimeric protein" or "fusion protein" as used herein refers to a protein formed by expression of a hybrid gene made by combining two or more gene sequences. Typically this is accomplished by cloning one or more cDNA sequences into an expression vector in frame with an existing gene.

Suitable systems for production of recombinant proteins include but are not limited to prokaryotic systems *(e.g., Escherichia coli),* yeast *(e.g., Saccaromyces cerevisiae, Pichia pastoris),* insect cells *(e.g.,* baculovirus), mammalian cells *(e.g.,* Chinese hamster ovary, HEK293), plants *(e.g.,* safflower), avian cells, amphibian cells, fish cells, and cell-free systems *(e.g.,* rabbit reticulocyte lysate).

As used herein, the term "vector" is used in reference to nucleic acid molecules that transfer DNA segment(s) from one cell to another. The term "vehicle" is sometimes used interchangeably with "vector."

The term "expression vector" as used herein refers to a recombinant DNA molecule containing a desired coding sequence and appropriate nucleic acid sequences necessary for the expression of the operably or operationally linked coding sequence in a particular host organism. Nucleic acid sequences necessary for expression in prokaryotes usually include a promoter, an operator (optional), and a ribosome-binding site, often along with other sequences. Eukaryotic cells are known to utilize promoters, enhancers, and termination and polyadenylation signals.

As used herein, the term "host cell" refers to any eukaryotic or prokaryotic cell, *e.g.*, bacterial cells such as *E. coli,* yeast cells, mammalian cells, avian cells, amphibian cells, plant cells, fish cells, and insect cells. Insect host cells include, but are not limited to *Spodoptera frugiperda* ovarian cell lines SF9 and SF21 and the *Trichoplusia ni* egg-derived cell line High Five. Host cells may be located *in vitro* or *in vivo*. For example, host cells may be located in a cell culture or in a transgenic animal. The recombinant polypeptides can be expressed by either transgenic technology or by infection with chimeric virus. The standard method to introduce foreign genes into plants is the well characterized single-stranded RNA virus, tobacco mosaic virus.

The term "transformation" as used herein refers to the introduction of foreign DNA into prokaryotic or eukaryotic cells. Transformation maybe accomplished by a variety of means known to the art including calcium phosphate-DNA coprecipitation, DEAE-dextran-mediated transfection, polybrene-mediated transfection, electroporation, microinjection, liposome fusion, lipofection, protoplast fusion, retroviral infection, and biolistics.

Preferred embodiments and advantages of the invention will be apparent from the following detailed description and figures.
- Figure 1: shows examples of preferred IgY receptor fusion proteins comprising human IgE constant domains
- Figure 2: shows examples of preferred IgY receptor fusion proteins comprising human IgG constant domains
- Figure 3: shows examples of preferred IgY receptor fusion proteins comprising human IgA constant domains
- Figure 4: shows examples of preferred IgY receptor fusion proteins comprising human IgM constant domains
- Figure 5: shows the structure of construct I (A) and II (B)
- Figure 6: shows the analysis by SDS-PAGE (A) and immunoblot (B) of a dimeric CHIR-AB1 construct (construct I)
- Figure 7: shows an analysis of the specificity of construct I (CHIR-huIgE Fc) using immobilized antibodies and anti-human IgE AP conjugate as detection antibody.
- Figure 8: shows the immunoreactivity of preformed complexes of recombinant anti-lysozyme IgY and construct I.
- Figure 9: shows the immunoreactivity of preformed complexes of recombinant anti-lysozyme IgY, construct I, and anti-human-IgE-AP-conjugate.
- Figure 10: shows the immunoreactivity of preformed complexes of polyclonal anti-TNP-BSA IgY and construct I.
- Figure 11: shows quantitation of polyclonal IgY using construct I (CHIR-IgEDC1His) as capture reagent and anti-IgE-AP as detection antibody.
- Figure 12: shows the immunoreactivity of construct II (CHIR-muIgG1 Fc) with immobilized polyclonal IgY and anti-mouse IgG AP conjugate as a detection antibody

While IgY antibodies provide important advantages for diagnostic applications, there are also major limitations to the use of IgY. Most important, there is a need in the field for high-affinity reagents for the detection of antigen- or allergen-specific IgY antibodies which can be adapted to detection procedures for mammalian immunoglobulins.

Avian high-affinity IgY Fc receptors include molecules of avian origin which are capable of specifically binding FcY with a K_{D} ≤ 50 nM. In one specific embodiment, receptor molecules encoded by the chicken Ig-like receptor (CHIR) multigene family localized in the leukocyte receptor complex (LRC) are employed. Chicken Ig-like receptors consist of single extracellular Ig domain, a basic transmembrane charge, and a cytoplasmic immunoreceptor tyrosine-based inhibitory motif (ITIM). The chicken LCR sequence is incomplete, but already 103 genes highly related to each other have been identified (Laun K, et al., PLoS Genet 2: e73, 2006). The CHIR-AB1, which has been cloned recently (Viertlboeck BC, et al., Proc. Natl. Acad. Sci, USA 104: 11718-11723, 2007), binds FcY with a K_{D} of 5.4 nM and IgY with a K_{D} of 27 nM. Other receptor molecules encoded by the CHIR multigene family have been shown to bind IgY with a K_{D} as low as 25 nM (Viertlboeck, BC, et al., J Immunol 182: 6985-6992, 2009a). In another specific embodiment, IgY FcR-related receptor molecules encoded by chicken genes located on a chromosomal region distinct from the LCR and FcR gene clusters are employed. One IgY receptor molecule encoded by these genes, designated gallus gallus FcR (ggFcR), has been shown to consist of four extracellular C2-set Ig domains, a transmembrane region containing arginine as a positively charged amino acid, and a short cytoplasmic tail (Viertlboeck BC, et al., J Immunol 182: 1533-1540, 2009b).

Mammalian constant immunoglobulin domains include the constant domain of A and κ light chains and the heavy chain constant domains of the five mammalian Ig isotypes, IgG, IgM, IgA, IgE and IgD. The heavy chains that define the different isotypes are designated by the lower-case greek letters γ, µ, α, ε and δ. With regard to the heavy chain constant domains of IgG, varying IgG antibody subclasses comprising varying γ heavy chain constant domains, known to the person skilled in the art, are present in different mammalian species including, but not limited to, humans, mice, rats, rabbits, goats, and sheep. For example, the constant domains of γ heavy chains in humans include the constant domains of IgG1, IgG2, IgG3 and IgG4. In mice, the constant domains of γ heavy chains include the constant domains of IgG1, IgG2a, IgG2b and IgG3, and in rats, the constant domains of γ heavy chains include the constant domains of IgG1, IgG2a, IgG2b and IgG2c. Sequence differences between immunoglobulin heavy chains cause the various isotypes to differ in several characteristic respects. These include the number and location of interchain disulfide bonds, the number of attached oligosaccharide moieties, the number of constant domains, and the length of the hinge region. IgM and IgE heavy chains contain an extra constant domain that replaces the hinge region found in γ, α and 5 chains. The structural characteristics of the various heavy chain constant domains of mammalian Ig isotypes are known to the person skilled in the art.

A mammalian constant domain in the context of the invention refers to an immunoglobulin domain that has a homology of at least 50%, preferably at least 70%, more preferably at least 80% or at least 90%, most preferred at least 95% amino acid homology to the corresponding wild-type mammalian domain. Thus, e.g., one, two, three or more amino acids can be deleted, inserted or substituted. In particular, conservative substitutions are contemplated. The boundaries between the domains are preferably defined as by the conserved domain retrieval tool (Geer LY, et al., Genome Res. 12(10): 1612-23, 2002), however, they can also be shifted.

In the following preferred fusion proteins are described. In the context of the invention "chimeric IgY Fc receptor construct" refers to constructs comprising the extracellular portion of an avian high-affinity IgY Fc receptor or the extracellular portion of an avian FcR-related molecule and mammalian constant immunoglobulin domains. The IgY Fc receptor molecule can be fused to the N- or C-terminus of mammalian constant immunoglobulin domains. The mammalian constant immunoglobulin domains may form monomeric constructs or dimeric constructs. The structural characteristics of many mammalian constant immunoglobulin domains and their capabilities to form monomeric or dimeric constructs have been published and are known to the person skilled in the art.

Constructs comprising truncated mammalian heavy chains provide a lower molecular mass than those comprising complete mammalian heavy chains, which in many cases has been shown to be associated with increased expression rates. Instead of the conventional domains, fragments or variants thereof that do not inhibit proper folding of the constructs can be used. The boundaries between the domains, and thus the species origin of fragments at these boundaries can also be varied, as long as proper folding of the constructs is ensured.

The avian high-affinity IgY Fc receptor of preferred chimeric IgY Fc receptor constructs is selected from a group including the extracellular portion of FcY receptor molecules encoded by the chicken Ig-like receptor (CHIR) multigene family localized in the leukocyte receptor complex (LRC), and the extracellular portion of IgY FcR-related receptor molecules encoded by chicken genes located on a chromosomal region distinct from the LCR and FcR gene clusters. Most preferred chimeric IgY Fc receptor constructs comprise the extracellular portion of the FcY receptor CHIR-AB1.

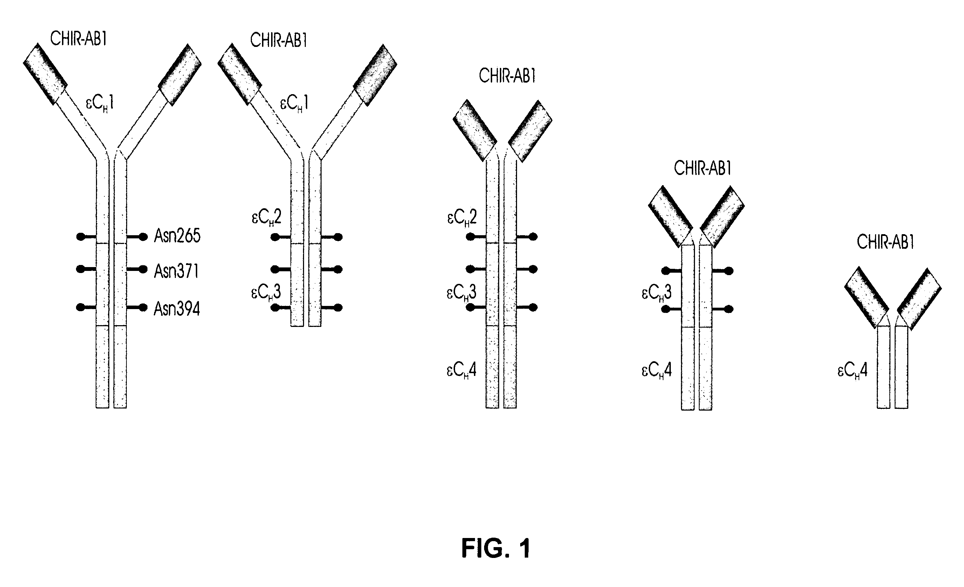

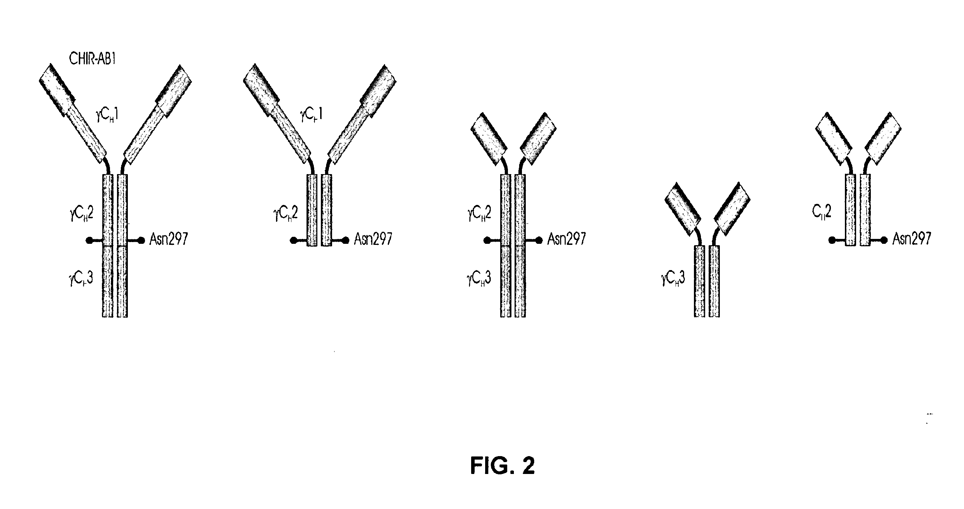

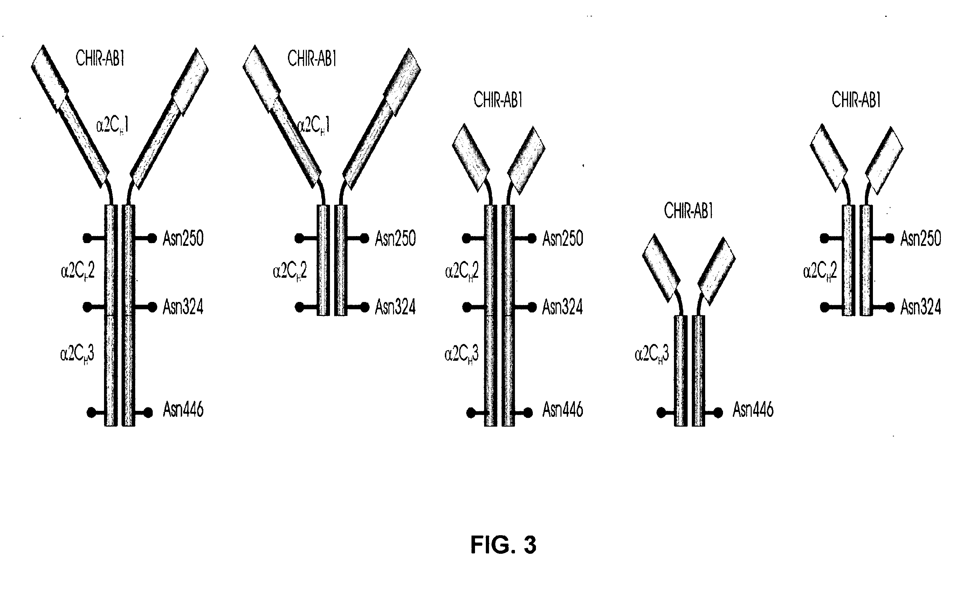

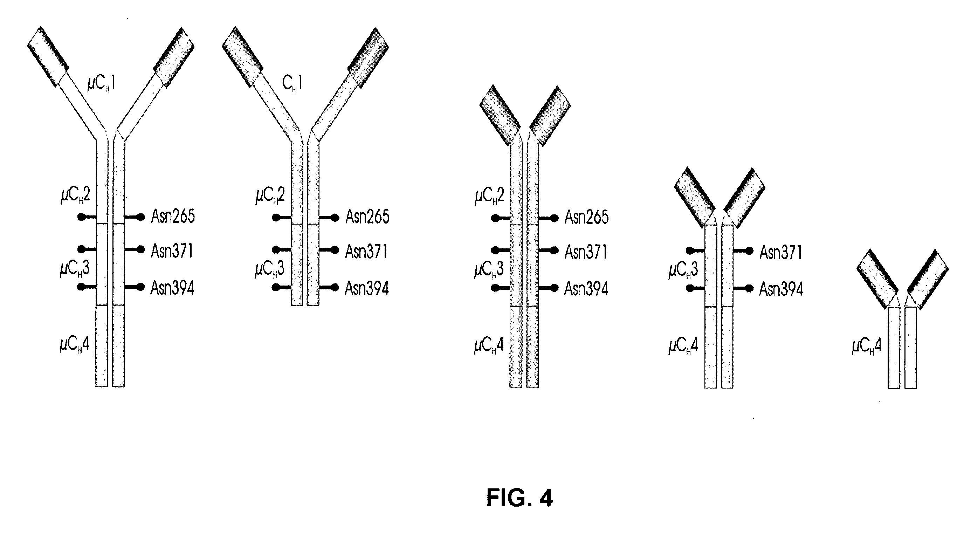

Examples of chimeric IgY Fc receptor constructs comprising human constant immunoglobulin domains are shown in figures 1-4. For dimeric IgY Fc receptor constructs comprising human IgE constant heavy chain domains, domains are selected from the group consisting of C_{H}1, C_{H}2, C_{H}3 and C_{H}4 IgE domains. The dimeric construct can comprise two, three or four IgE heavy chain constant domains. Preferably, the dimeric construct comprises a) C_{H}1, C_{H}2, C_{H}3, C_{H}4, b) C_{H}1, C_{H}2, C_{H}3, c) C_{H}2, C_{H}3, C_{H}4, d) C_{H}3 and C_{H}4, and e) C_{H}4 IgE heavy chain constant domains. Structural analysis of human IgE revealed an asymmetrically bent conformation of IgE with the C_{H}2 domains forming highly bent structures at C_{H}2-C_{H}3 junctions (Wan T, et al., Nat. Immunol. 3, 681-686, 2002). Therefore, truncation of mammalian IgE heavy chains at the C_{H}2 domain is considered likely to fundamentally impair the folding and assembly process of IgE. For monomeric constructs, the IgE heavy chain domains are selected from the group consisting of C_{H}1, C_{H}3 and C_{H}4 IgE domains. Preferably, the monomeric construct comprises a) C_{H}1, C_{H}3, C_{H}4, b) C_{H}1, C_{H}3, c) C_{H}1, C_{H}4, d) C_{H}1, e) C_{H}3, C_{H}4, f) C_{H}3, and g) C_{H}4 IgE heavy chain constant domains.

For dimeric IgY Fc receptor constructs comprising human IgG constant heavy chain domains, domains are selected from the group consisting of C_{H}1, C_{H}2 and C_{H}3 IgG domains. The dimeric construct can comprise one, two or three IgG heavy chain constant domains of the four human IgG isotypes: IgG1, IgG2, IgG3, and IgG4. Preferably, the dimeric construct comprises a) C_{H}1, C_{H}2, C_{H}3 (all human IgG isotypes), b) C_{H}1 and C_{H}2 (all human IgG isotypes except IgG1), c) C_{H}2 and C_{H}3 (human IgG isotypes IgG2, IgG3 and IgG4), d) C_{H}2, and e) C_{H}4 (all human IgG isotypes) IgG heavy chain constant domains. For monomeric constructs, the IgG heavy chain domains are selected from the group consisting of of C_{H}1 and C_{H}4 IgG domains. Preferably, the monomeric construct comprises a) C_{H}1 and C_{H}3 (all human IgG isotypes except IgG1), b) C_{H}1 (all human IgG isotypes), and c) C_{H}3 (all human IgG isotypes) IgG heavy chain constant domains.

For dimeric IgY Fc receptor constructs comprising human IgA constant heavy chain domains, domains are selected from the group consisting of C_{H}1, C_{H}2 and C_{H}3 IgA domains. The dimeric construct can comprise one, two or three IgA heavy chain constant domains. Preferably, the dimeric construct comprises a) C_{H}1, C_{H}2, C_{H}3 b) C_{H}1 and C_{H}2 c) C_{H}2 and C_{H}3 and d) C_{H}2, and e) C_{H}3 IgA heavy chain constant domains. For monomeric constructs, the IgA heavy chain domains are selected from the group consisting of of C_{H}1 and C_{H}4 IgA domains. Preferably, the monomeric construct comprises a) C_{H}1 and C_{H}3 b) C_{H}1 and c) C_{H}3 IgA heavy chain constant domains.

For dimeric IgY Fc receptor constructs comprising human IgM constant heavy chain domains, domains are selected from the group consisting of C_{H}1, C_{H}2, C_{H}3 and C_{H}4 IgM domains. The dimeric construct can comprise one, two, three or four IgM heavy chain constant domains. Preferably, the dimeric construct comprises a) C_{H}1, C_{H}2, C_{H}3, C_{H}4, b) C_{H}1, C_{H}2, C_{H}3, c) C_{H}2, C_{H}3, C_{H}4, d) C_{H}3, C_{H}4, e) C_{H}2, and f) C_{H}4 IgM heavy chain constant domains. For monomeric constructs, the IgM heavy chain domains are selected from the group consisting of C_{H}1, C_{H}3 and C_{H}4 IgM domains. Preferably, the monomeric construct comprises a) C_{H}1, C_{H}3, C_{H}4, b) C_{H}1, C_{H}3, c) C_{H}1, C_{H}4, d) C_{H}1, e) C_{H}3, C_{H}4, f) C_{H}3, and g) C_{H}4 IgM heavy chain constant domains.

The chimeric IgY Fc receptor constructs comprise constant immunoglobulin domains derived from mice, rats, horses, dogs, cats and other mammalian animals (Hellman L, Eur J Immunol 23: 159-67, 1993; Marti E, et al, Vet Immun Immunpath 47: 363-7, 1995; Navarro P, et al, Mol Immun 32: 1-8, 1995; Patel M, et al, Immunogenetics 41: 282-6, 1995; Weber ER, et al, Vet Immun Immunpath 76: 299-308, 2000; Wagner B, et al, Immunogenetics 54: 353-64, 2002).

Other preferred chimeric IgY receptor constructs comprise the extracellular portion of an avian high-affinity IgY Fc receptor or the extracellular portion of an avian FcR-related molecule and mammalian constant immunoglobulin domains fused by an oligo- or polypeptide linker to provide increased flexibility.

Other preferred chimeric IgY receptor constructs are heterotetrameric constructs comprising four molecules of the extracellular portion of an avian high-affinity IgY Fc receptor or the extracellular portion of an avian FcR-related molecule, fused to light and heavy chain mammalian constant immunoglobulin domains, thereby increasing avidity of IgY Fc binding.

Other preferred chimeric IgY receptor constructs comprise the extracellular portion of an avian high-affinity IgY Fc receptor or the extracellular portion of an avian FcR-related molecule, mammalian constant immunoglobulin domains, and additional non-immunoglobulin oligo- or polypeptides. Such non-immunoglobulin oligo- or polypeptides may have various functions, including but not limited to affinity purification of the construct of invention.

In the following preferred nucleic acids are described. The present invention also provides nucleic acid sequences and vectors encoding the above listed constructs of the invention. To produce the constructs, one having ordinary skill in the art can prepare a DNA molecule encoding the construct using well known techniques. The coding sequence can be obtained from natural sources or synthesized or otherwise constructed using widely available starting materials by routine methods. In some embodiments, a DNA molecule that includes a nucleotide sequence that encodes a construct of the invention may be synthesized using the amino acid sequence information herein and the genetic code. When the coding DNA is prepared synthetically, advantage can be taken of known codon preferences of the intended host where the DNA is to be expressed. One having ordinary skill in the art can insert that DNA molecule into a commercially available expression vector for use in well known expression systems, employing well known methods and readily available starting materials.

The present invention also comprises nucleic acid sequences in which potential N-glycosylation sites of constant immunoglobulin domains are eliminated by site-directed mutation using well known techniques. Thereby, potential interaction of the mammalian constant immunoglobulin domains via their oligosaccharide chains with C-type lectins in human sera and other human body fluids can be reduced significantly. C-type lectins bind sugars in a calcium-dependent manner via highly conserved carbohydrate recognition domains (CRD). C-Type lectins are either produced as transmembrane proteins, e.g., on dendritic cells and Langerhans cells or secreted as soluble proteins. Examples of soluble C-type lectins include the mannan-binding lectin (MBL), the surfactant protein A (SP-A) and D (SP-D), and the conglutenins CL-46 and CL-43, all of wich belong to the collectin family (for a review, see Van de Wetering JK., et al., Eur. J. Biochem. 271, 1229-1249, 2004). MBL is secreted into the blood stream. The presence of substantial amounts of MBL in the small intestine suggests that this protein is acting as a humoral immune factor in the intestine, similar to secretory IgA. SP-A and SP-D are secreted at the luminal surface of pulmonary epithelial cells, and recently an increased expression of SP-D in the gastric mucosa during *Heliobacter pylori* infection has been reported, pointing to a role of the surfactant proteins in mucosal defense systems. The serum collectins conglutenin CL-46 and CL-43 have so far only be detected in bovidae, where the liver is their main site of production. The basic functional unit of collectins is a trimer, but the number of trimeric units per collectin molecule differs among the collectins. MBL and SP-A form octadecamers of six trimeric subunits, with their overall structure resembling a bouquet of flowers, whereas SP-D and the bovine conglutein proteins are assembled into dodecamers of four trimeric subunits. In addition, SP-D can form even higher-order multimers with a mass of several million kDa. The size of fully assembled collectins ranges from 13 nm for MBL to about 100 nm for SP-D. In order to recognize a variety of cell surface saccharides, collectins provide a broad monosaccharide specificity including mannose, galactose, and glucose. Although the K_{d} of the binding of a single CRD with a monosaccharide ligand is in the order of 10⁻³ M., the K_{d} of binding of higher-order multimers of collectins to polyvalent ligands is in the order of 10⁻⁸ to 10⁻¹¹ M. As a result, glycosylated mammalian constant immunoglobulin domains can be bound tightly via their oligosaccharide chains by collectins. For example, MBL has been shown to bind agalactosylated glycoforms of IgG (Malhotra R., et al. Nat. Med. 1, 237-243, 1995) and polymeric forms of serum IgA (Roos A., et al. J. Immunol. 167, 2861-2868, 2001). Utilizing glycosylated monoclonal capture antibodies in ELISA-type assays, the presence of soluble C-type lectins in sera of patients can lead to false positive results as described for RF and HAMA (Boscato LM, Stuart MC, Clin Chem 34, 27-33, 1988). Since many C-type lectins such as MBL form oligomers of trimeric subunits, C-type lectins bound to glycosylated monoclonal capture antibodies are likely to provide additional binding sites for glycosylated detection antibodies or a glycosylated chimeric fusion construct of the invention which could result in false positive data. This limitation can be reduced b y utilizing a chimeric fusion construct of the invention comprising mammalian constant immunoglobulin domains in which potential N-glycosylation sites are eliminated.

The structural characteristics of the various constant domains of mammalian immunoglobulin isotypes including the putative N-glycosylation sites have been published and are known to the person skilled in the art. Mutation of N-glycosylation sites of the sequence Asn-Xaa-Ser/Thr can include one or two amino acid residues of each site. For example, the asparagine (Asn) in the glycosylation site(s) can be exchanged against any other amino acid, preferably against glutamine (Gln) (Elbein AD et al., 1991, Trends Biotechnol. 9(10):346-352). Alternatively, in a mutated glycosylation site, the serine (Ser) can be exchanged against another amino acid, preferably against alanine (Ala), and the threonine (Thr) can be exchanged preferably against valine (Val). Most preferably, all N-glycosylation of sites of the mammalian constant immunoglobulin domains fused to the extracellular portion of an avian high-affinity IgY Fc receptor or the extracellular portion of an avian FcR-related molecule are mutated. Techniques for site-directed mutations of nucleic acids are known to the person skilled in the art.

In the following the expression of constructs is described in detail. A large number of vector-host systems known in the art may be used. Possible vectors include, but are not limited to, plasmids or modified viruses, but the vector system must be compatible with the host cell used. Potential host-vector sytems include, but are not limited to mammalian cell systems infected with virus (e.g., vaccinia virus, adenovirus, etc.), insect cell systems infected with virus (e.g.; baculovirus), microorganisms such as yeast containing yeast vectors, or bacteria transformed with bacteriophages (e.g., lambda derivatives), DNA, plasmid DNA (e.g., various pBR322 derivatives), or cosmid DNA. The expression elements of vectors vary in their strenghts and specificities. Depending on the host-vector system utilized, any one of a number of suitable transcription and translation elements may be used. In an alternative embodiment, the chimeric fusion protein of the invention is expressed chromosomally, after integration after the coding sequence by recombination. In this regard, any of a number of amplification systems may be used to achieve high levels of stable gene expression.

The present invention further relates to a host cell comprising said expression vector. This host cell includes, but is not limited to a bacterial cell, a yeast cell, a mammalian cell, an insect cell, or a plant cell. Alternatively, a cell-free expression system may be used. The development and optimization of specific methods as well as approaches to overcome a variety of problems associated with any of these expression systems have been published in many articles (for a review, see Schmidt M and Hoffman DR, Int. Arch. Allergy Immunol. 2002; 128: 264-270).

In one embodiment, bacterial expression systems (e.g E. coli) are utilized for the production of large quantities of the chimeric fusion protein, preferably for the production of large quantities of monomeric chimeric fusion proteins. To avoid potential misfolding and segregation of said polypeptides into insoluble aggregates as inclusion bodies upon overproduction, an expression system that secretes the chimeric fusion protein into the periplasmatic space can be used to improve solubility of said polypeptides. The periplasmatic space is an oxidizing environment that contains enzymes catalyzing disulfide bonds. Co-overproduction of protein disulfide isomerases may be applied to enhance disulfide bond formation. Other proper folding factors such as peptidyl-prolyl-*cis*/*trans* isomerise also have been demonstrated to be helpful in improving solubility. To avoid potential problems in translation, arginine codons AGA and AGG which are rare and inefficiently translated in prokaryotes, can be replaced by site-directed mutagenesis. Alternatively, special strains of E. coli with extra copies of the necessary tRNA genes suitable to circumvent this problem can be selected.

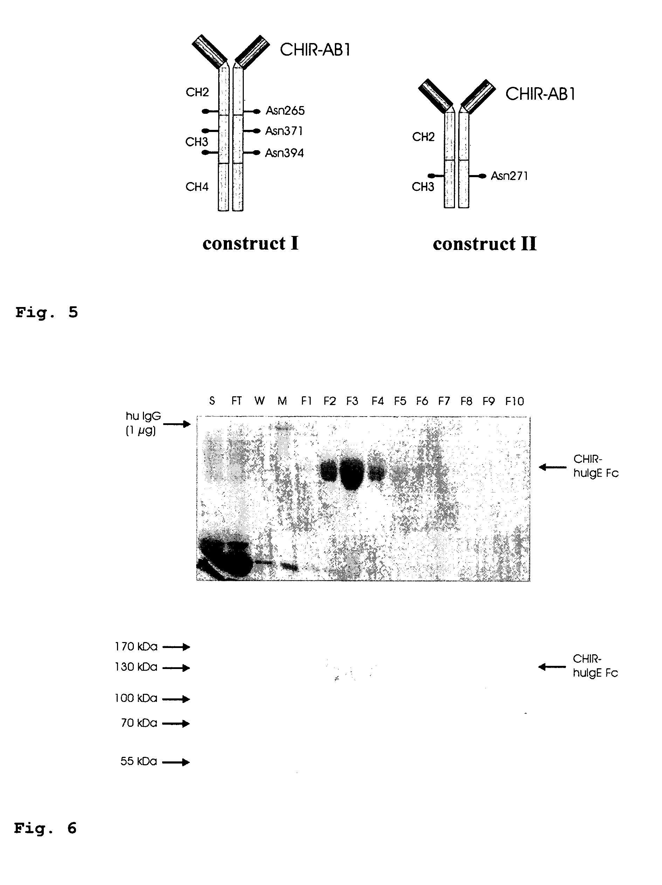

In another embodiment, eukaryotic expression systems including mammalian, yeast, plant, and insect cells are utilized preferably for the production of chimeric fusion proteins requiring complex disulfide bond formation including homodimeric constructs. Eukaryotic cells provide a variety of processing mechanisms in the endoplasmatic reticulum (ER) including chaperone-assisted folding. Mammalian cells such as CHO or HEK293 cells are preferred for the production of the chimeric fusion protein of the invention. Among the many types of yeast the methylotrophic *Pichia pastoris* is preferred due to its capability to produce recombinant proteins in high yield. In addition, both intact plants and cell lines are also suitable for the production of the chimeric fusion protein. The chimeric fusion protein can be expressed by either transgenic technology or by infection with chimeric virus. The standard method to introduce foreign genes into plants is the well characterized single-stranded RNA virus, tobacco mosaic virus. Furthermore, insect cells utilizing the baculovirus system can be applied for the production of the chimeric fusion protein. The particulars for the construction of expression systems suitable for the desired host are known to those skilled in the art. As shown in figure 6, HEK293 cells proved to be capable of expressing and secreting the chimeric fusion protein of the invention as an immunoreactive fusion protein. Thus, the present invention also relates to a method of expressing the chimeric fusion protein of the invention comprising the extracellular portion of an avian high-affinity IgY Fc receptor and mammalian constant immunoglobulin domains in eukaryotic cells.

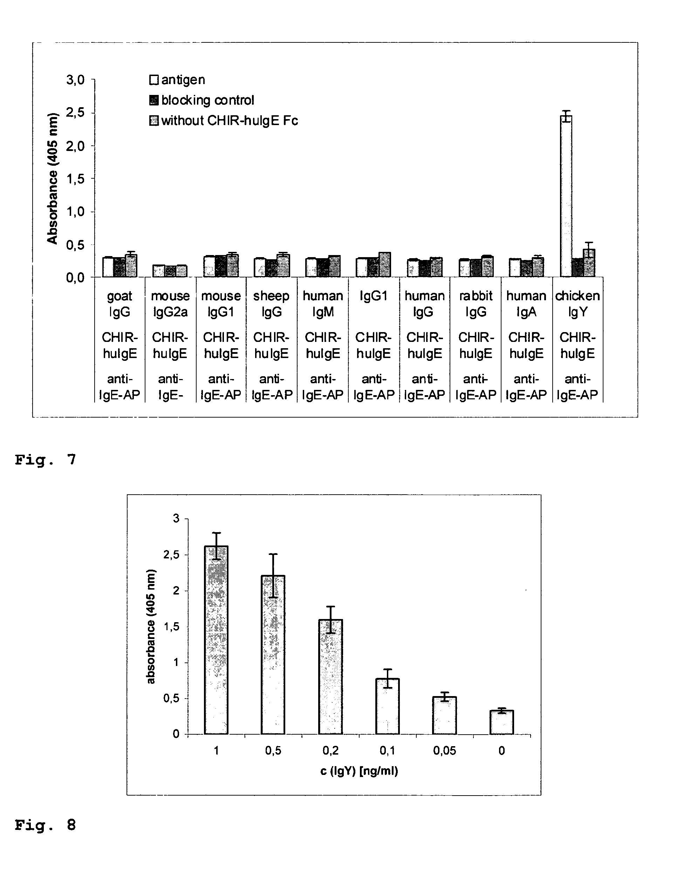

In the following biotechnological and diagnostic application are described in detail. As described above IgY antibodies offer great opportunities for diagnostic applications and the chimeric fusion protein of the invention provides the necessary adapter molecule that allows to detect and quantify IgY antibodies by procedures developed for mammalian immunoglobulins. The chimeric fusion protein of the invention allows sensitive detection of avian IgY since those avian IgY Fc receptor molecules utilized for the construction of a chimeric fusion protein are capable of binding FcY with high affinity. For example, the CHIR-AB1 receptor binds FcY with a K_{D} of 5.4 nM (Viertlboeck BC, et al., Proc. Natl. Acad. Sci, USA 104: 11718-11723, 2007). Utilizing dimeric fusion constructs the apparent affinity for IgY may be even higher due to avidity effects. Those avian IgY Fc receptor molecules utilized for the construction of a chimeric fusion protein provide also a high degree of specificity for IgY as demonstrated for a chimeric fusion protein comprising the CHIR-AB1 receptor (see figure 7).

In one embodiment of the invention, the chimeric fusion protein of the invention is applied as reagent for the detection of antigen- or allergen-bound IgY antibodies in immunological assays including, but not limited to ELISA-type assays, immunoblots and immunodotblotting. The chimeric fusion protein allows to detect and quantify antigen- or allergen-bound IgY antibodies by established and inexpensive procedures developed for mammalian immunoglobulins. Furthermore, the large variety of commercially available detection procedures for mammalian antibodies allows to modify the detection procedure according to the needs of a particular assay, e.g. with regard to sensitivity.

In another embodiment, the chimeric fusion protein is used together with antigen- or allergen-specific IgY antibodies as a positive control reagent for immunoassays developed for mammalian immunoglobulins. Preferred assay examples include, but are not limited to ELISA-type assays designed for the detection and quantification of human antibodies, e.g. allergen-specific IgE or IgG4 antibodies and autoimmune disease-related IgG, IgM or IgA antibodies. Since the availability of positive control sera for such assays can be extremely limited, the chimeric fusion protein provides a valuable tool to establish an alternative positive control. Typically, assays designed for the detection and quantification of antigen- or allergen-specific antibodies in human sera are performed in two steps. First, serum antibodies are incubated with immobilized antigens or allergens. Thereafter, bound serum antibodies are detected with labeled anti-human immunoglobulin antibodies (secondary antibodies). Utilizing antigen- or allergen-specific IgY antibodies as positive control antibodies, the assay can be performed also in two steps. First, immobilized antigens or allergens are incubated with an appropriate mixture of antigen- or allergen-specific IgY antibodies and the chimeric fusion protein comprising appropriate human constant immunoglobulin domains. Thereafter, bound complexes of antigen- or allergen-specific IgY and the chimeric fusion protein are detected with labeled anti-human immunoglobulin antibodies (secondary antibodies). A preferred appropriate mixture of antigen- or allergen-specific IgY antibodies and the chimeric fusion protein contains both components at a concentration that allows to complex all or the vast majority of antigen- or allergen-specific IgY antibodies with the chimeric fusion protein. By varying the concentration of antigen- or allergen-specific IgY antibodies in the sample, the positive control can be adjusted according to the needs of each assay. If an additional amplication step is required to achieve sufficient sensitivity, detection and quantification of antigen- or allergen-specific antibodies in human sera is performed in three or more steps. Utilizing antigen- or allergen-specific IgY antibodies as positive control antibodies for such assays, both the antigen- or allergen-specific IgY antibodies and the chimeric fusion protein may be incubated with the immobilized antigens or allergens in subsequent steps.

In a specific embodiment, the chimeric fusion protein of the invention and allergen-specific IgY antibodies are utilized as positive control antibodies in assays for the detection and quantification of allergen-specific IgE antibodies in human sera. For this application the chimeric fusion protein comprises the extracellular portion of an avian high-affinity IgY Fc receptor or the extracellular portion of an avian FcR-related molecule with a high-affinity IgY binding capability, and at least one human IgE constant immunoglobulin domain, preferably human IgE constant immunoglobulin domains that are capable of mediating the formation of a dimeric construct. In a typical assay designed for the detection and quantification of allergen-specific IgE antibodies in human sera, allergen-specific serum IgE antibodies are incubated with immobilized allergens. Thereafter, bound IgE antibodies are detected with labeled anti-human IgE antibodies (secondary antibodies). Utilizing allergen-specific IgY antibodies as positive control antibodies, the assay can be performed also in two steps. First, immobilized allergens are incubated with an appropriate mixture of allergen-specific IgY antibodies and the chimeric fusion protein comprising human IgE constant immunoglobulin domains. Thereafter, bound complexes of allergen-specific IgY and the chimeric fusion protein are detected with labeled anti-human IgE antibodies (secondary antibodies).

In another specific embodiment, the chimeric fusion protein of the invention comprises at least two constant immunoglobulin domains derived from different antibody isotypes of the same mammalian species. Thereby, one chimeric fusion construct can be used for the detection of different antibody isotypes, depending on the number of constant domains from different antibody isotypes in the chimeric fusion construct. Preferred assay examples include, but are not limited to ELISA-type assays suitable for the detection and quantification of different isotypes of human antibodies, e.g. assays for allergen-specific IgE and IgG4 antibodies or assays for different isotypes of autoantibodies with the same specificity for an autoimmune disease-related antigen. For example, in allergy diagnostic determination of the allergen-specific IgE:IgG4 ratio in peripheral blood is useful to assess the success of any specific immunotherapy. A chimeric fusion protein comprising both human IgE and IgG4 constant immunoglobulin domains can be used to establish an IgY-based positive control for both assays. Such a universal construct provides an important advantage due to reduced production costs. Similar considerations apply to assays for antibodies in human serum against autoimmune disease-related antigens. For example, in some cases the phospholipid-antibody syndrome is associated with the presence of autoantibodies against cardiolipin. Since such autoantibodies can be IgG, IgM or IgA antibodies, a chimeric fusion protein comprising human IgG, IgM and IgA constant immunoglobulin domains can be used to establish an IgY-based positive control for all three assays with a single construct.

In still another specific embodiment, the chimeric fusion proteins of the invention comprises at least two constant immunoglobulin domains derived from different mammalian species. Thereby, one chimeric fusion construct can be used for the detection of antibodies from different mammalian species, depending on the number of constant immunoglobulin domains from different mammalian species in the chimeric fusion construct. Preferred assay examples include, but are not limited to ELISA-type assays suitable for the detection and quantification of allergen-specific antibodies in different animals. For example, *in vitro* assays for the diagnosis of allergy in race horses and pets such as dogs and cats become more and more popular, but positive control sera for such assays are almost unavailable. A chimeric fusion protein comprising appropriate constant immunoglobulin domains from different animals represents an attractive alternative to establish an IgY-based positive control for these assays.

In another embodiment, the chimeric fusion protein is used together with antigen- or allergen-specific IgY antibodies as standardization reagent for immunoassays developed for mammalian immunoglobulins. Preferred assay examples include, but are not limited to ELISA-type assays designed for the detection and quantification of human antibodies, e.g. allergen-specific IgE or IgG4 antibodies and autoimmune disease-related IgG, IgM or IgA antibodies. For such assays reliable standardization reagents are not available since the composition and concentration of antigen-or allergen-specific antibodies in individual positive control sera varies significantly. Currently, human pool sera are utilized, but the varying characteristics of each individual serum and the limited quantity of available sera pose serious problems. Antigen- or allergen-specific IgY antibodies and the chimeric fusion protein comprising appropriate human constant immunoglobulin domains provide a valuable alternative to establish reliable standardization reagents for such assays. Recombinant expression of the chimeric fusion protein guarantees reproducibility and same is true for monoclonal IgY antibodies. Although polyclonal IgY suffer from the known disadvantages of polyclonal antibodies including varying specificities and affinities of different antibody batches, they provide also some very important advantages. They are inexpensive and are easy to obtain in relatively large quantities. A chicken usually lays about 280 eggs per year, and each egg yolk contains 100-150 mg IgY. As a result, large quantities of antigen- or allergen-specific IgY antibodies can be collected and stored, possibly sufficient for more than a decade. Therefore, the chimeric fusion protein together with polyclonal antigen- or allergen-specific IgY provide an affordable tool to establish an alternative standardization reagent. The IgY-based standardization reagent can be used as described for the IgY-based positive control reagent above. However, for standardization purposes the amount of antigen- or allergen-specific IgY and the chimeric fusion protein needs to be defined in each standardization sample to guarantee reliable reproducibility. In order to allow classification of patient's sera according to the titer of antigen- or allergen-specific antibodies, standardization samples containing varying amounts of antigen- or allergen-specific IgY and the chimeric fusion protein are useful. Such standardization samples can be applied separately according to needs of a given assay or together to generate a standard curve.

In a specific embodiment, the chimeric fusion protein of the invention is applied as reagent for the detection of IgY-based standardization reagents in immunological assays designed for the determination of mammalian antibodies. In another specific embodiment of the invention, the chimeric fusion protein of the invention comprising at least two constant immunoglobulin domains derived from different antibody isotypes of the same mammalian species, is applied as reagent for the detection of IgY-based standardization reagents in immunological assays designed for the determination of different mammalian antibody isotypes of the same mammalian species. In still another specific embodiment of the invention, the chimeric fusion protein of the invention comprising at least two constant immunoglobulin domains derived from different mammalian species, is applied as reagent for the detection of IgY-based standardization reagents in immunological assays designed for the determination of mammalian antibodies from different mammalian species.

In another specific embodiment, the chimeric fusion protein of the invention is used in combination with antigen-or allergen-specific IgY antibodies to generate artificial mammalian sera for positive control experiments and/or standardization of immunoassays designed for the determination of mammalian antibodies. In another specific embodiment of the invention, the chimeric fusion protein of the invention comprising at least two constant immunoglobulin domains derived from different antibody isotypes of the same mammalian species, is used in combination with antigen- or allergen-specific IgY antibodies to generate artificial mammalian sera for positive control experiments and/or standardization of immunoassays designed for the determination of different mammalian antibody isotypes of the same mammalian species. In still another specific embodiment of the invention, the chimeric fusion protein of the invention comprising at least two constant immunoglobulin domains derived from different mammalian species, is used in combination with antigen- or allergen-specific IgY antibodies to generate artificial mammalian sera for positive control experiments and/or standardization of immunoassays designed for the determination of mammalian antibodies from different mammalian species. By the addition of different amounts of antigen-or allergen-specific IgY antibodies and an appropriate chimeric fusion protein to normal human sera from healthy volunteers, these sera are converted to standardized artificial pool sera mimicking sera from sick patients. Standardized artificial pool sera mimicking sera from sick mammalian animals are prepared accordingly. Such sera can be prepared reproducibly and are available in unlimited quantities and, thereby, represent reliable reference sera.

Another aspect of the present invention is to utilize the chimeric fusion protein together with antigen- or allergen-specific IgY antibodies for the preparation of standardized solutions, in which human serum is replaced by a synthetic medium suitable for positive control experiments and standardization of immunoassays. Synthetic media suitable for the present invention include but are not restricted to those containing buffer-diluted normal human sera from healthy volunteers, buffer-diluted sera of any other mammalian species, a selected number of human serum components, a selected number of serum components of any other mammalian species, non-serum proteins of human origin or those derived from any other species, recombinant proteins, synthetic polypeptides, and those containing non-proteinaceous components. Non-proteinaceous components include but are not restricted to the large variety of buffer systems known to one having ordinary skill in the art, amino acid residues, oligopeptides, and natural as well as synthetic oligo/polysaccharides.

It is another aspect of the present invention to use chimeric fusion proteins of the invention as tools for affinity purification of avian IgY antibodies. The avian high-affinity IgY Fc receptor of preferred chimeric IgY Fc receptor constructs is selected from a group including the extracellular portion of FcY receptor molecules encoded by the chicken Ig-like receptor (CHIR) multigene family localized in the leukocyte receptor complex (LRC), and the extracellular portion of IgY FcR-related receptor molecules encoded by chicken genes located on a chromosomal region distinct from the LCR and FcR gene clusters. Most preferred chimeric IgY Fc receptor constructs comprise the extracellular portion of the FcY receptor CHIR-AB1. The mammalian constant immunoglobulin domains of the chimeric fusion proteins serve for immobilization onto a solid phase. Immobilization techniques include but are not limited to covalent immobilization by established techniques known to the person skilled in the art, covalent derivatization of the constant domains with an affinity ligand such as biotin and subsequent interaction with covalently immobilized corresponding affinity component such as streptavidin, and interaction of the constant domains with covalently immobilized protein A and protein G. For affinity purification of avian IgY chimeric fusion proteins provide an important advantage over non-fused IgY Fc receptor molecules, since the extracellular portion of preferred IgY Fc receptor molecules is relatively small and covalent immobilization of non-fused molecules is likely to impair their capability as capture reagent due to steric hindrance problems. IgY is a bulky ligand and high affinity binding to preferred IgY Fc receptor molecules requires unrestricted interaction of all involved amino acid residues.

It is still another aspect of the present invention to provide chimeric fusion proteins of the invention as tools for quantitation of avian IgY antibodies. Potential assay formats for quantitation of IgY include, but are not limited to two step ELISA procedures. For example, Chicken IgY can be captured by an immobilized chimeric fusion protein of the invention and captured IgY can be detected with an anti-chicken-IgY(Fc)-AP-conjugate as described in example 11. The avian high-affinity IgY Fc receptor of preferred chimeric IgY Fc receptor constructs for this application is selected from a group including the extracellular portion of FcY receptor molecules encoded by the chicken Ig-like receptor (CHIR) multigene family localized in the leukocyte receptor complex (LRC), and the extracellular portion of IgY FcR-related receptor molecules encoded by chicken genes located on a chromosomal region distinct from the LCR and FcR gene clusters. Most preferred chimeric IgY Fc receptor constructs comprise the extracellular portion of the FcY receptor CHIR-AB1. The mammalian constant immunoglobulin domains of the chimeric fusion proteins serve for immobilization onto a solid phase.

### EXAMPLE 1: Amplification of the extracellular domain of the avian leukocyte receptor CHIR-AB1

The avian leukocyte receptor CHIR-AB1 extracellular domain (CHIR-AB1-ecd) was synthesized from cDNA derived from chicken (*Gallus domesticus*) splenocytes. CHIR-AB1-ecd was amplified using one PCR primer containing a Smi I site (GATC ATTTAAATGTGTCCAGTGT CTG CCC CAA CCC TCC CTG TC, SEQ ID NO:1) and another primer containing an Asc I site (GATC GGCGCGCC ATG GGA CCA CTC CTT GGC ACC, SEQ ID NO:2). Utilizing Pwo-Polymerase (PeqLab, Erlangen, Germany) in combination with a mastercycler gradient (Eppendorf, Hamburg, Germany), following protocol was applied for PCR amplifications: 1x 90 s 95°C, 30x (20 s 95°C, 30 s 60°C, 45 s 72°C), 1x 5 min 72°C.

### EXAMPLE 2: Amplification of human ε constant domains

Human immunoglobulin constant domains were synthesised from cDNA derived from human peripheral mononuclear cells isolated from whole blood. The human ε heavy chain domains 2-4 were amplified using the PCR primers containing an Asc I site (GATC GGCGCG CC C ACC GTG AAG ATC TTA C, SEQ ID NO:3) and an Xba I site(GATC TCTAGA TCA ATG GTG GTG ATG GTG TTT ACC GGG ATT TAC AGA CAC CG, SEQ ID NO:4) providing a 5x His-Tag. Amplification by PCR was performed as described in example 1.

### EXAMPLE 3: Amplification of murine γ1 constant domains

Murine immunoglobulin constant domains were synthesised from cDNA derived from murine splenocytes. The murine γ 1 heavy chain domains 2-3 were amplified using the PCR primers containing an Asc I site (GATC GGCGCGCC T AAG CCT AAG CCT TGC ATA TGT ACA GTC C, SEQ ID NO:5) and an Xba I site (GAT CTC TAG ATC AGT GGT GGT GGT GTT TAC CAG GAG AGT GGG A, SEQ ID NO:6) providing a 4x His-Tag. Amplification by PCR was performed as described in example 1.

### EXAMPLE 4: Cloning of a chimeric fusion protein comprising CHIR-AB1-ecd and human ε constant domains (construct I)

For convenient expression of the constructs a set of modular cassettes containing a leader sequence, constant immunoglobulin domains, and restriction sites for the incorporation of CHIR-AB1-ecd was generated. The human ε heavy chain domains 2-4 were inserted via Asc I and Xba I into the mammalian expression vector pcDNA3.1-zeo (Invitrogen life technologies, Karlsruhe, Germany) containing a modified human heavy chain leader sequence vH3-64 which provides an internal Smi I site (ATG GAA TTG GGG CTG AGC TGG GTT TTC CTT GTT GCT ATA TTT AAA TGT GTC CAGTGT, SEQ ID NO: 7) inserted via Nhe I (GAT CGC TAG CAT GGA ATT GGG GCT GAG CTG G, Seq ID No: 8). Introduction of CHIR-AB1-ecd into the vector was performed via the Smi I site at the N-terminus (SEQ ID NO:1) and an Asc I site at the C-terminus (SEQ ID NO:2) of the receptor fragment by PCR. Subsequently, the DNA was ligated into the vector pcDNA3.1-zeo (Invitrogen life technologies, Karlsruhe, Germany) containing the signal sequence and the particular constant regions. Construct I is shown in figure 5, left side. Evident therefrom is the homodimeric receptor Fc fusion protein CHIR-huIgE Fc (left). Indicated are the extracellular domain of the chicken leukocyte receptor CHIR-AB1 (upper slanted part), the particular immunoglobulin Fc regions (lower upright parts), and the Fc N-glycosylation sites (black dots).

### EXAMPLE 5: Cloning of a chimeric fusion protein comprising CHIR-AB1-ecd and murine γ1 constant domains (construct II)

The modular cassettes of example 4 containing a leader sequence, constant immunoglobulin domains, and restriction sites for the incorporation of CHIR-AB1-ecd were used. The murine γ 1 heavy chain domains 2-3 were inserted via Asc I and Xba I into the mammalian expression vector pcDNA3.1-zeo (Invitrogen life technologies, Karlsruhe, Germany) containing a modified human heavy chain leader sequence vH3-64 which provides an internal Smi I site (ATG GAA TTG GGG CTG AGC TGG GTT TTC CTT GTT GCT ATA TTT AAA TGT GTC CAGTGT, SEQ ID NO: 7) inserted via Nhe I (GAT CGC TAG CAT GGA ATT GGG GCT GAG CTG G, Seq ID No: 8). Introduction of CHIR-AB1-ecd into the vector was performed via the Smi I site at the N-terminus (SEQ ID NO:1) and an Asc I site at the C-terminus (SEQ ID NO:2) of the receptor fragment by PCR. Subsequently, the DNA was ligated into the vector pcDNA3.1-zeo (Invitrogen life technologies, Karlsruhe, Germany) containing the signal sequence and the particular constant regions. Construct II, the homodimeric receptor Fc fusion protein CHIR-muIgG1 Fc, is shown in figure 5, right side. The Parts indicated correspond with those described in Example 4.

### EXAMPLE 6: Eukaryotic expression of construct I (example 4) and construct II (example 5)

HEK-293 cells (ATCC number CRL-1573) were cultivated in DMEM (Dulbecco's modified Eagle Medium) supplemented with 10% (v/v) heat-inactivated fetal calf serum (FCS), 100 IU/ml penicillin, and 100 µg/ml streptomycin. Tissue culture reagents were obtained from Invitrogen life technologies (Karlsruhe, Germany). HEK-293 cells growing in DMEM supplemented with 10% (v/v) FCS were transfected with 2 µg of the particular expression vector using PEI (polyethylenimine, Sigma, Taufkirchen, Germany). Stable transfectants then were selected in DMEM supplemented with 10% (v/v) FCS and 100 µg/ml of zeocin (Invitrogen life technologies, Karlsruhe, Germany).

For expression of the constructs, transfected cells were grown for 3 days as an adhesion culture. The constructs secreted by transfected HEK-293 cells were purified from the culture medium by affinity chromatography using Ni-NTA-agarose (Qiagen, Hilden, Germany) according to the manufacturer's recommendations.

As an example, figure 6 (SDS/PAGE and immunoblotting analyses of recombinant IgY formats; S supernatant, FT flowthrough, W wash, M Marker (Page Ruler Prestained, Fermentas, St. Leon-Roth, Germany), F elution fraction) shows the analysis by SDS-PAGE (A) and immunoblot (B) of a dimeric CHIR-AB1 construct (construct I) comprising human IgE constant domains C_{H}2-C_{H}4, expressed and purified as described. For immunoblot analysis, purified recombinant CHIR-huIgE Fc was separated by SDS-PAGE using a tris/glycin-buffered 7.5% polyacrylmide gel. The proteins were transferred onto a nitrocellulose membrane by western blotting. After incubation for 90 min with 1% (w/v) BSA (Sigma, Taufenstein, Germany) diluted in phosphate-buffered saline at room temperature on a rocker platform, the blot membrane was rinsed 3 times with PBS and further incubated with 10 ml of anti-human-IgE-AP conjugate (diluted in phosphate-buffered saline supplemented with 1% (w/v) BSA (Sigma, Taufenstein, Germany) for 60 min at room temperature on a rocker platform. The membrane was rinsed again 3 times each with 0.1% (v/v) TPBS and PBS and CHIR-huIgE Fc was visualized by addition of 10 ml of a BCIP/NBT substrate solution diluted in 100 mM Tris-HCl, 5 mM MgCl₂, pH 9.5 (Sigma, Taufenstein, Germany) until bands became visible.

### EXAMPLE 7: Specificity of construct I comprising CHIR-AB1-ecd and human IgE constant domains.

Polyclonal chicken IgY purified from egg yolk, polyclonal goat IgG (E0432, DAKO A/S, Denmark), monoclonal mouse IgG1 (F-1888, Sigma), monoclonal mouse IgG2a (A-5691, Sigma ImmunoChemicals), polyclonal sheep IgG (code 682711, ICN biomedicals), polyclonal rabbit IgG, polyclonal human IgM (SigmaImmunoChemicals), polyclonal human IgG, monoclonal human IgG1 (Nimotuzumab, Astra Zeneca), polyclonal human IgA (Sigma) were diluted in phosphate-buffered saline (PBS; final concentration 25 µg/ml), applied to microtiter plates at 4° C overnight, and blocked with 1% (w/v) BSA at room temperature for 2 h. After incubation of the microtiter plates for 90 min at room temperature on a rocker platform, the wells were rinsed 3 times each with Tween (0.1% v/v)-phosphate-buffered saline (TPBS) and PBS and further incubated with 100 µl each of cell culture supernatant construct I (CHIR-huIgE Fc; diluted in 10% (w/v) FCS) for 60 min at room temperature on a rocker platform. The wells were rinsed again 3 times each with 0.1% (v/v) TPBS and PBS and further incubated with 100 µl each of polyclonal anti-human-IgE-AP conjugate (diluted 1:1000 in 10% FCS, BD Biosciences, Heidelberg, Germany) for 60 min at room temperature on a rocker platform. The wells were rinsed again 3 times each with 0.1% (v/v) TPBS and PBS and bound antibodies were visualized by addition of 75 µl of a 4-nitrophenyl disodium orthophosphate substrate solution (Sigma, Taufenstein, Germany). Absorbance was determined at 405 nm after 20 min of incubation. Data of the experiment are shown in figure 7 (analysis of the specificity of construct I (CHIR-huIgE Fc) using immobilized antibodies and anti-human IgE AP conjugate as detection antibody). Negative controls were performed by omission of construct I, positive controls were performed by using an anti-chicken IgG (Fc) AP conjugate (diluted 1:2000 in phosphate buffered saline supplemented with 10% FCS, Rockland Immunochemicals, Gilbertsville, PA, USA).

### EXAMPLE 8: Immunoreactivity of preformed complexes of recombinant IgY and construct I comprising CHIR-AB1-ecd and human IgE constant domains

Purified hen egg lysozyme was diluted in phosphate-buffered saline (10 µg/ml; Sigma, Taufenstein, Germany), applied to microtiter plates at 4° C overnight, and blocked with 10% (v/v) FCS at room temperature for 2 h. After incubation of the microtiter plates for 90 min at room temperature on a rocker platform, the wells were rinsed 3 times each with Tween (0.1% v/v)-phosphate-buffered saline (TPBS) and PBS and further incubated for 60 min at room temperature on a rocker platform with 100 µl each of preformed complexes of recombinant anti-hen egg lysozyme IgY (HyHEL-IgY; Greunke K, et al, J Biotechnol 124(2): 446-56, 2006) and construct I comprising CHIR-AB1-ecd and human IgE constant domains. The preformed complexes were generated by adding varying amounts of purified HyHEL-IgY (as indicated in Fig. 8) to cell culture supernatant containing CHIR-huIgE Fc (construct I). The wells were rinsed again 3 times each with 0.1% (v/v) TPBS and PBS and further incubated with 100 µl each of polyclonal anti-human-IgE-AP conjugate (diluted 1:1000 in phosphate-buffered saline supplemented with 10% FCS, BD Biosciences, Heidelberg, Germany) for 60 min at room temperature on a rocker platform. The wells were rinsed again 3 times each with 0.1% (v/v) TPBS and PBS and bound antibodies were visualized by addition of 75 µl of a 4-nitrophenyl disodium orthophosphate substrate solution (Sigma, Taufenstein, Germany). Absorbance was determined at 405 nm after 20 min of incubation. Data of the experiment are shown in Fig. 8.

### EXAMPLE 9: Immunoreactivity of preformed complexes of recombinant IgY, construct I comprising CHIR-AB1-ecd and human IgE constant domains, and anti-IgE-AP

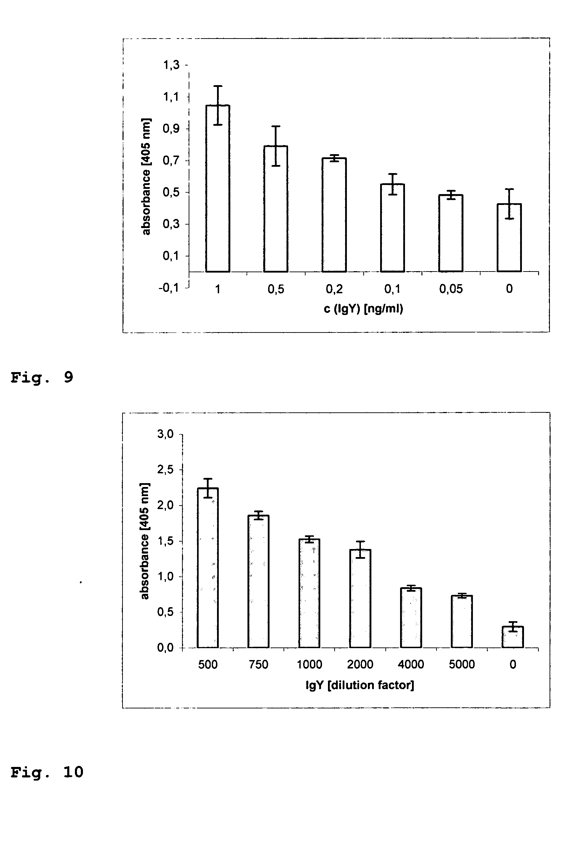

Purified hen egg lysozyme was diluted in phosphate-buffered saline (10 µg/ml PBS; Sigma, Taufenstein, Germany), applied to microtiter plates at 4° C overnight, and blocked with 10% (v/v) FCS at room temperature for 2 h. After incubation of the microtiter plates for 90 min at room temperature on a rocker platform, the wells were rinsed 3 times each with Tween (0.1% v/v)-phosphate-buffered saline (TPBS) and PBS and further incubated for 60 min at room temperature on a rocker platform with 100 µl each of preformed complexes of recombinant anti-hen egg lysozyme IgY (HyHEL-IgY; Greunke K, et al, J Biotechnol 124(2): 446-56, 2006), construct I comprising CHIR-AB1-ecd and human IgE constant domains, and polyclonal anti-human-IgE-alkaline phosphatase (AP)-conjugate (BD Biosciences, Heidelberg, Germany). The preformed complexes were generated by adding varying amounts of purified HyHEL-IgY (as indicated in Fig. 9) to cell culture supernatant containing CHIR-huIgE Fc (construct I) and anti-human-IgE-AP-conjugate (diluted 1:1000 in PBS supplemented with 10% (w/v) FCS). The wells were rinsed again 3 times each with 0.1% (v/v) TPBS and PBS and bound antibodies were visualized by addition of 75 µl of a 4-nitrophenyl disodium orthophosphate substrate solution (Sigma, Taufenstein, Germany). Absorbance was determined at 405 nm after 20 min of incubation (data see Fig. 9).

### EXAMPLE 10: Immunoreactivity of preformed complexes of polyclonal IgY and construct I comprising CHIR-AB1-ecd and human IgE constant domains

Bovine serum albumin (1.0 mg BSA/ml, Cohn Fraction V, Sigma, Taufenstein, Germany) diluted in 50 mM Na₂HCO₃, pH 8.5, was haptenylated by addition of a 20 molar excess of 2,4,6-trinitrophenyl benzene sulfonic acid (TNBS, picrylsulfonic acid, Sigma, Taufenstein, Germany), and further dialyzed against phosphate-buffered saline. TNP-BSA (100 µg/ml diluted in phosphate-buffered saline) was then applied to microtiter plates at 4° C overnight and blocked with 10% (v/v) FCS at room temperature for 2 h. After incubation of the microtiter plates for 90 min at room temperature on a rocker platform, the wells were rinsed 3 times each with Tween (0.1% v/v)-phosphate-buffered saline (TPBS) and PBS and further incubated for 60 min at room temperature on a rocker platform with 100 µl each of preformed complexes of polyclonal anti-TNP-BSA IgY and construct I comprising CHIR-AB1-ecd and human IgE constant domains. The preformed complexes were generated by adding varying dilutions (as indicated in Fig. 10) of polyclonal anti-TNP-BSA IgY purified from egg yolk of chicken (18 mg/ml stock solution in phosphate-buffered saline) previously immunized with TNP-BSA following standard protocols (Eurogentec Deutschland GmbH, Koeln, Germany), to cell culture supernatant containing CHIR-huIgE Fc (construct I). The wells were rinsed again 3 times each with 0.1% (v/v) TPBS and PBS and further incubated with 100 µl each of polyclonal anti-human-IgE-AP conjugate (diluted 1:1000 in phosphate-buffered saline supplemented with 10% FCS, BD Biosciences, Heidelberg, Germany) for 60 min at room temperature on a rocker platform. Bound antibodies were visualized by addition of 75 µl of a 4-nitrophenyl disodium orthophosphate substrate solution (Sigma, Taufenstein, Germany). Absorbance was determined at 405 nm after 20 min of incubation. Data of the experiment are shown in Fig. 10.

### EXAMPLE 11: Quantitation of chicken IgY using construct I as capture reagent

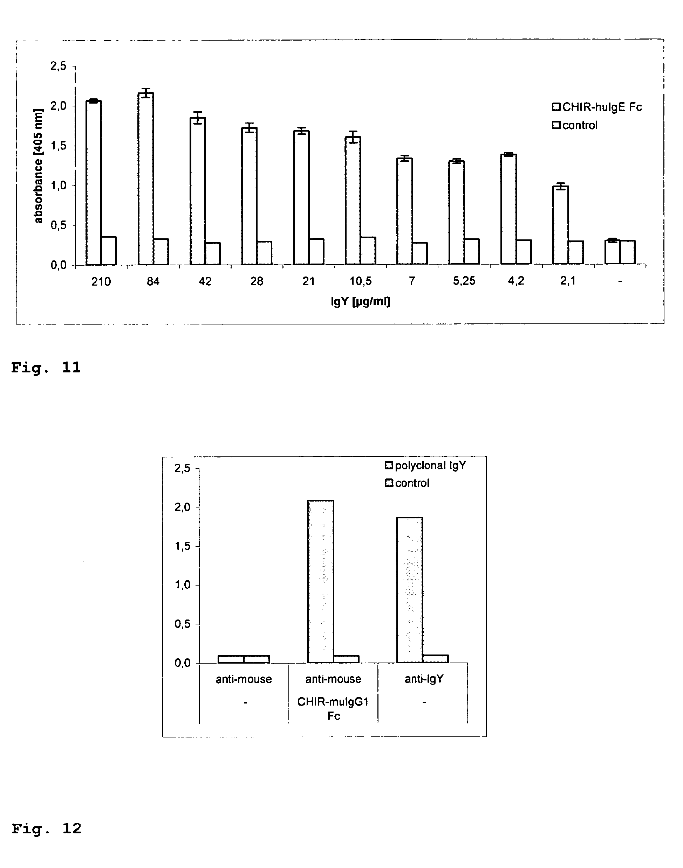

Purified construct I (CHIR-huIgE Fc, 100 µg/ml phosphate buffered saline) was applied to microtiter plates at 4° C overnight and blocked with 10% (v/v) FCS in phosphate buffered saline at room temperature for 2 h. The wells were rinsed 3 times each with Tween (0.1% v/v)-PBS (TPBS) and PBS and further incubated for 60 min at room temperature on a rocker platform with varying amounts (as indicated in Fig. 11) of purified chicken IgY from egg yolk (stock solution: 21 mg/ml PBS) in 100 µl of phosphate buffered saline supplemented with 10% v/v FCS each. The wells were rinsed again 3 times each with 0.1% (v/v) TPBS and PBS and further incubated for 60 min at room temperature on a rocker platform with 100 µl each of monoclonal anti-chicken-IgY(Fc)-AP conjugate (Rockland Immuno-chemicals, Gilbertsville, PA, USA) diluted 1:2000 in phosphate buffered saline supplemented with 10% (v/v) FCS. The wells were rinsed again 3 times each with 0.1% (v/v) TPBS and PBS and bound antibodies were visualized by addition of 75 µl of a 4-nitrophenyl disodium orthophosphate substrate solution (Sigma, Taufenstein, Germany). Absorbance was determined at 405 nm after 20 min of incubation. Data of the experiment are shown in Fig. 11.

### EXAMPLE 12: Immunoreactivity of construct II comprising CHIR-AB1-ecd and murine IgG1 constant domains.

Purified IgY from hen egg yolk was diluted in phosphate buffered saline (100 µg/ml), applied to microtiter plates at 4° C overnight, and blocked with phosphate buffered saline supplemented with 10% (v/v) FCS in phosphate buffered saline at room temperature for 2 h. After incubation of the microtiter plates for 90 min at room temperature on a rocker platform, the wells were rinsed 3 times each with Tween (0.1% v/v)-phosphate-buffered saline (TPBS) and PBS and further incubated for 60 min at room temperature on a rocker platform with 100 µl each of cell culture supernatant containing construct II (CHIR-muIgG1 Fc). The wells were rinsed again 3 times each with 0.1% (v/v) TPBS and PBS and further incubated with 100 µl each of polyclonal anti-mouse-IgG-AP-conjugate (diluted 1:5000 in phosphate-buffered saline supplemented with 10% FCS, Sigma, Germany) for 60 min at room temperature on a rocker platform. The wells were rinsed again 3 times each with 0.1% (v/v) TPBS and PBS and bound antibodies were visualized by the addition of 75 µl of a 4-nitrophenyl disodium orthophosphate substrate solution (Sigma, Taufenstein, Germany). Absorbance was determined at 405 nm after 20 min of incubation. Data of the experiment are shown in Fig 12. Negative controls were performed by omission of construct II (CHIR-huIgE Fc), positive controls were performed by using an anti-chicken-IgY(Fc)-AP conjugate (diluted 1:2000 in phosphate buffered saline supplemented with 10% (v/v) FCS, Rockland Immunochemicals, Gilbertsville, PA, USA).

### Sequences

SEQ ID NO:1
   GATC ATTTAAATGTGTCCAGTGT CTG CCC CAA CCC TCC CTG TC
SEQ ID NO:2
   GATC GGCGCGCC ATG GGA CCA CTC CTT GGC ACC
SEQ ID NO:3
   GATC GGCGCG CC C ACC GTG AAG ATC TTA C
SEQ ID NO:4 
GATC TCTAGA TCA ATG GTG GTG ATG GTG  TTT ACC GGG ATT TAC AGA
CAC CG
SEQ ID NO:5
   GATC GGCGCGCC T AAG CCT AAG CCT TGC ATA TGT ACA GTC C
SEQ ID NO:6
   GAT CTC TAG ATC AGT GGT GGT GGT GTT TAC CAG GAG AGT GGG A
SEQ ID NO: 7 
ATG GAA TTG GGG CTG AGC TGG GTT TTC CTT GTT GCT ATA TTT AAA
TGT GTC CAGTGT
SEQ ID NO: 8
   GAT CGC TAG CAT GGA ATT GGG GCT GAG CTG G
SEQ ID NO: 9 (Nucleic acid sequence of CHIR-huIgE Fc, underlined: restriction sites SmiI, SgsI, XbaI) 
atggaattggggctgagctgggttttccttgttgctatatttaaatgtgtccagtgtctgc
cccaaccctccctgtcgctgcaccccagccagggggtgtccctgggggacactgtcaccct
gcgctgccacctgccccgcatggctgcctgggtccagctctggctcaatggaactctgaga
tttgacaaggaaaaagacaaggagcaggatgcagctgagttctcctttgctgtcgcaaacc
tggaggacgccgggacatatcagtgtcggtaccaggtgtcagagccactgtggacatcaaa
tcagagtgaccccgtggagctggtgctgacaggtgccaaggagtggtcccatggcgcgccc
accgtgaagatcttacagtcgtcctgcgacggcggcgggcacttcccccgaccatccagc
tcctgtgcctcgtctctgggtacaccccagggactatcaacatcacctggctggaggacgg
gcaggtcatggacgtggacttgtccaccgcctctaccacgcaggagggtgagctggcctcc
acacaaagcgagctcaccctcagccagaagcactggctgtcagaccgcacctacacctgcc
aggtcacctatcaaggtcacacctttgaggacagcaccaagaagtgtgcagattccaaccc
gagaggggtgagcgcctacctaagccggcccagcccgttcgacctgttcatccgcaagtcg
cccacgatcacctgtctggtggtggacctggcacccagcaaggggaccgtgaacctgacct
ggtcccgggccagtgggaagcctgtgaaccactccaccagaaaggaggagaagcagcgcaa
tggcacgttaaccgtcacgtccaccctgccggtgggcacccgagactggatcgagggggag
acctaccagtgcaggtgacccacccccacctgcccagggccctcatgcggtccacgacca
agaccagcggcccgcgtgctgccccggaagtctatgcgtttgcgacgccggagtggccggg
gagccgggacaagcgcaccctcgcctgcctgatccagaacttcatgcctgaggacatctcg
gtgcagtggctgcacaacgaggtgcagctcccggacgcccggcacagcacgacgcagcccc
gcaagaccaagggctccggcttcttcgtcttcagccgcctggaggtgaccagggccgaatg
ggagcagaaagatgagttcatctgccgtgcagtccatgaggcagcgagcccctcacagacc
gtccagcgagcggtgtctgtaaatcccggtaaacaccatcaccaccattgatctaga
SEQ ID NO: 10 (Amin acid sequence of CHIR-huIgE Fc, underlined L and K are the first and last amino acids of the mature protein, *italics:* modified human immunoglobulin vH-63 signal sequence, **bold:** extracellular domain of CHIR-AB1) 
MELGLSWVFLVAIFKCVQCLPQPSLSLHPSQGVSLGDTVTLRCHLPRMAAWVQLWLNGTLR
FDKEKDKEQDAAEFSFAVANLEDAGTYQCRYQVSEPLWTSNQSDPVELVLTGAKEWSHGAP
TVKILQSSCDGGGHFPPTIQLLCLVSGYTPGTINITWLEDGQVMDVDLSTASTTQEGELAS
TQSELTLSQKHWLSDRTYTCQVTYQGHTFEDSTKKCADSNPRGVSAYLSRPSPFDLFIRKS
PTITCLVVDLAPSKGTVNLTWSRASGKPVNHSTRKEEKQRNGTLTVTSTLPVGTRD
WIEGETYQCRVTHPHLPRALMRSTTKTSGPRAAPEVYAFATPEWPGSRDKRTLACLIQNFM
PEDISVQWLHNEVQLPDARHSTTQPRKTKGSGFFVFSRLEVTRAEWEQKDEFICRAVHEAA
SPSQTVQRAVSVNPGKHHHHHH
SEQ ID NO: 11(nucleic acid sequence of CHIR-muIgG1 Fc, underlined: restriction sites SmiI, SgsI, XbaI) 
atggaattggggctgagctgggttttccttgttgctatatttaaatgtgtccagtgtctgc
cccaaccctccctgtcgctgcaccccagccagggggtgtccctgggggacactgtcaccct
gcgctgccacctgccccgcatggctgcctgggtccagctctggctcaatggaactctgaga
tttgacaaggaaaaagacaaggagcaggatgcagctgagttctcctttgctgtcgcaaacc
tggaggacgccgggacatatcagtgtcggtaccaggtgtcagagccactgtggacatcaaa
tcagagtgaccccgtggagctggtgctgacaggtgccaaggagtggtcccatggcgcgcct
aagcctaagccttgcatatgtacagtcccagaagtatcatctgtcttcatcttccccccaa
agcccaaggatgtgctcaccattactctgactcctaaggtcacgtgtgttgtggtagacat
 
cagcaaggatgatcccgaggtccagttcagctggtttgtagatgatgtggaggtgcacaca
gctcagacgcaaccccgggaggagcagttcaacagcactttccgctcagtcagtgaacttc
ccatcatgcaccaggactggctcaatggcaaggagttcaaatgcagggtcaacagtgcagc
tttccctgcccccatcgagaaaaccatctccaaaaccaaaggcagaccgaaggctccacag
gtgtacaccattccacctcccaaggagcagatggccaaggataaagtcagtctgacctgca
tgataacagacttcttccctgaagacattactgtggagtggcagtggaatgggcagccagc
ggagaactacaagaacactcagcccatcatggacacagatggctcttacttcgtctacagc
aagctcaatgtgcagaagagcaactgggaggcaggaaatactttcacctgctctgtgttac
atgaggcctgcacaaccaccatactgagaagagcctctcccactctcctggtaaacacca
ccaccactgatctagagatc
SEQ ID NO: 12 (amino acid sequence of CHIR-muIgG1 Fc,

underlined L and K are the first and last amino acids of the
mature protein, italics: modified human immunoglobulin vH-63
signal sequence, bold: extracellular domain of CHIR-AB1)
MELGLSWVFLVAIFKCVQCLPQPSLSLHPSQGVSLGDTVTLRCHLPRMAAWVQLWLNGTLR
FDKEKDKEQDAAEFSFAVANLEDAGTYQCRYQVSEPLWTSNQSDPVELVLTGAKEWSHGAP
KPKPCICTVPEVSSVFIFPPKPKDVLTITLTPKVTCVVVDISKDDPEVQFSWFVDDVEVHT
AQTQPREEQFNSTFRSVSELPIMHQDWLNGKEFKCRVNSAAFPAPIEKTISKTKGRPKAPQ
VYTIPPPKEQMAKDKVSLTCMITDFFPEDITVEWQWNGQPAENYKNTQPIMDTDGSYFVYS
KLNVQKSNWEAGNTFTCSVLHEGLHNHHTEKSLSHSPGKHHHH-SRD

### List of references

Arnon TI et al., J. Mol. Biol. 381, 1012-1024, 2008.
Baatrup G et al., Scand J Immunol 23, 397-406, 1986.
Boscato LM et al., Clin chem 32, 1491-1495, 1986.
Boscato LM et al., Clin Chem 34, 27-33, 1988.
Campbell RD et al., Biochem J 189, 6780, 1980.
Chambers RE et al., Ann Clin Biochem 24, 520-524, 1987.
Elbein AD, Tibtech 9, 346-352, 1991.
Geer LY et al., Genome Res. 12, 1619-1623, 2002.
Greunke K et al., J. Biotechnol. 124, 446-456, 2006.
Hellman L, Eur. J. Immunol. 23, 159-167, 1993.
Hoffman WL et al., J Immunol Methods 198, 67-77, 1996.
Horton J et al., J Invest Dermatol 85, 96-99, 1984.
Johnson PM et al., Clin Immunol Immunopathol 6, 414-430, 1976.
Kapyaho K et al., Scand J Clin Lab Invest 49, 211215, 1989.
Kricka LJ, Clin Chem 45, 942-956, 1999.
Larsson A et al., J Immunol Methods 119, 103-109, 1989; Clin Chem 37, 411-414, 1991; Hybridoma 11, 33-39, 1992; J Immunol Methods 156, 79-83, 1992.
Laun K et al., PLOS Genet. 2, e73.
Leslie GA et al., J. Exp. Med. 130, 1337-1352.
Lindahl TL et al., Thromb Haemost 68, 221-225, 1992.
Malhotra R et al., Nature Med. 1, 237-243.
Marti E et al., Vet. Immun. Immunpath. 47, 363-367, 1995.
Miller GW et al., Proc Natl Acad Sci U S A 72, 418422, 1975.
Navarro P et al., Mol. Immunol. 32, 1-8, 1995.
Patel M et al., Immunogenet. 41, 282-286, 1995.
Roos A et al., J. of Immunol. 167, 2861-2868.
Schmidt M et al., Allergy Immunol. 128, 264-270, 2002.
Sun S et al., Rapid Commun Mass Spectrom 15, 708-712, 2001.
Van de Wetering JK et al.,Eur. J. Biochem. 271, 1229-1249, 2004.
Van de Winkel JG et al., Immunol Today 14, 215-221, 1993.
Viertlboeck BC et al., PNAS 104, 11718-23, 2007; J. Immunol. 182, 6985-92, 2009; J. Immunol. 182, 1533-40, 2009.
Wagner B et al., Immunogenet. 54, 353-364, 2002.
Wan T et al., Nat. Immunol. 3, 681-686, 2002.
Weber ER et al., Vet Immun. Immunpath. 76, 299-308, 2000.
Whaley, K. Measurement of complement. In Methods in complement for clinical immunologists (K. Whaley, ed.), p. 77-139. Churchill Livingstone, Edinburgh, United Kingdom, 1985.

## Claims

1. Isolated recombinant chimeric construct comprising at least one peptide comprised in the constant region of a mammalian immunoglobulin selected from the group consisting of human IgE domains C_{H}1, C_{H}2, C_{H}3 and C_{H}4, IgM domains C_{H}1, C_{H}2, C_{H}3 and C_{H}4, human IgA domains C_{H}1, C_{H}2 and C_{H}3, human IgG2 domains C_{H}1, C_{H}2 and C_{H}3, human IgG3 domains C_{H}1, C_{H}2 and C_{H}3, human IgG4 domains C_{H}1, C_{H}2 and C_{H}3, human IgG1 domains C_{H}1, C_{H}2 and C_{H}3, and constant immunoglobulin domains derived from mice, rats, horses, dogs, cats and other mammalian animals, and an avian component selected from the group consisting of the extracellular portion of an avian high-affinity IgY Fc receptor or the extracellular portion of an avian FcR-related molecule wherein the peptide and the avian component are covalently bound to each other, and wherein the construct is capable of detecting antigen- or allergen-specific IgY antibodies by utilizing detection procedures for mammalian immunoglobulins.

2. Construct according to claim 1, wherein at least two different peptides comprised in the constant region of a mammalian immunoglobulin are comprised, wherein the different peptides preferably are comprised in different isotypes of the mammalian immunoglobulin of the same mammalian species, or are comprised in different immunoglobulins from different mammalian species.

3. Construct according to claim 1 or 2, wherein the construct is monomeric or homodimeric.

4. Construct according to any of the claims 1 to 3, comprising the extracellular portion of the avian leukocyte receptor CHIR-AB1.

5. Construct according to any of the claims 1 to 4, wherein the construct is a protein or a peptide.

6. Isolated nucleic acid coding for a construct according to any of the claims 1 to 5.

7. Expression vector comprising as a coding sequence a nucleic acid according to claim 6 and a promoter wherein the nucleic acid is under the control of the promoter.

8. Method for producing a construct according to any of the claims 1 to 5, wherein a cell, preferably a prokaryotic or eukaryotic cell, in particular a mammalian cell, is transformed with the expression vector of claim 6, wherein the cell is cultured for a predetermined time period and under predetermined conditions, and wherein during or after culturing the construct is isolated either from the culturing supernatant or from the cell.

9. Use of a construct according to any of the claims 1 to 5 for the immunological detection of IgY antibodies.

10. Use of a construct according to any of the claims 1 to 5 for the detection of IgY based standardization reagents in immunoassays for the determination of mammalian antibodies.

11. Kit comprising the following components:
a) a construct according to any of the claims 1 to 5,
b) antigen- or allergen-specific IgY antibodies,
c) optionally, washing solutions and buffer solutions,
d) a detector reagent binding to the construct, wherein the components a), b) and/or d) may be included in separate packages or in one single package.

12. Host cell, in particular bacterial cell, yeast cell, mammalian cell, insect cell, or plant cell comprising the expression vector of claim 7.

## Patentansprüche

1. Isoliertes rekombinantes chimäres Konstrukt umfassend zumindest ein Peptid, enthalten in der konstanten Region eines Säugetier-Immunglobulins ausgewählt aus der Gruppe bestehend aus humanen IgE-Domänen C_{H}1, C_{H}2, C_{H}3 und C_{H}4, IgM-Domänen C_{H}1, C_{H}2, C_{H}3 und C_{H}4, humanen IgA-Domänen C_{H}1, C_{H}2 und C_{H}3, humanen IgG2-Domänen C_{H}1, C_{H}2 und C_{H}3, humanen IgG3-Domänen C_{H}1, C_{H}2 und C_{H}3, humanen IgG4-Domänen C_{H}1, C_{H}2 und C_{H}3, humanen IgG1-Domänen C_{H}1, C_{H}2 und C_{H}3 und konstanten Immunglobulin-Domänen abgeleitet von Mäusen, Ratten, Pferden, Hunden, Katzen und anderen Säugetieren, und eine Vogel-Komponente ausgewählt aus der Gruppe bestehend aus dem extrazellulären Teil eines Vogel-IgY-Fc-Rezeptors mit hoher Affinität oder dem extrazellulären Teil eines FcR-verwandten Vogel-Moleküls, wobei das Peptid und die Vogel-Komponente kovalent aneinander gebunden sind, und wobei das Konstrukt in der Lage ist, antigen-oder allergen-spezifische IgY-Antikörper unter Verwendung von Erfassungsverfahren für Säugetier-Immunglobuline zu erfassen.

2. Konstrukt gemäß Anspruch 1, wobei zumindest zwei verschiedene Peptide, die in der konstanten Region eines Säugetier-Immunglobulins enthalten sind, vorgesehen sind, wobei die verschiedenen Peptide bevorzugt in verschiedenen Isotypen des Säugetier-Immunglobulins der gleichen Säugetier-Spezies enthalten sind oder in verschiedenen Immunglobulinen von verschiedenen Säugetier-Spezies enthalten sind.

3. Konstrukt gemäß Anspruch 1 oder 2, wobei das Konstrukt monomer oder homodimer ist.

4. Konstrukt gemäß einem der Ansprüche 1 bis 3, welches den extrazellulären Teil des Vogel-Leukozyten-Rezeptors CHIR-AB1 umfasst.

5. Konstrukt gemäß einem der Ansprüche 1 bis 4, wobei das Konstrukt ein Protein oder ein Peptid ist.

6. Isolierte Nukleinsäure, welche für ein Konstrukt gemäß einem der Ansprüche 1 bis 5 kodiert.

7. Expressionsvektor, welcher als Kodiersequenz eine Nukleinsäure gemäß Anspruch 6 und einen Promoter umfasst, wobei die Nukleinsäure unter Kontrolle des Promoters steht.

8. Verfahren zur Herstellung eines Konstrukts gemäß einem der Ansprüche 1 bis 5, wobei eine Zelle, bevorzugt eine prokaryotische oder eukaryotische Zelle, insbesondere eine Säugetier-Zelle, mit dem Expressionsvektor gemäß Anspruch 6 transformiert wird, wobei die Zelle über eine vorgegebene Zeit und unter vorgegebenen Bedingungen kultiviert wird, und wobei während oder nach der Kultivierung das Konstrukt entweder aus dem Kulturüberstand oder aus der Zelle isoliert wird.

9. Verwendung eines Konstrukts gemäß einem der Ansprüche 1 bis 5 zur immunologischen Erfassung von IgY-Antikörpern.

10. Verwendung eines Konstrukts gemäß einem der Ansprüche 1 bis 5 zur Erfassung von IgY-basierten Standardisierungs-Reagenzien in Immunassays zur Bestimmung von Säugetier-Antikörpern.

11. Kit, welcher die folgenden Komponenten umfasst:
a) ein Konstrukt gemäß einem der Ansprüche 1 bis 5,
b) antigen- oder allergen-spezifische IgY-Antikörper,
c) optional Waschlösungen und Pufferlösungen,
d) ein Detektorreagens, das an das Konstrukt bindet,
wobei die Komponenten a), b) und/oder d) in separaten Paketen oder in einem einzigen Paket enthalten sein können.

12. Wirtszelle, insbesondere Bakterien-Zelle, Hefe-Zelle, Säugetier-Zelle, Insekten-Zelle oder Pflanzen-Zelle, welche den Expressionsvektor gemäß Anspruch 7 umfasst.

## Revendications

1. Construction chimérique recombinante isolée comprenant au moins un peptide compris dans la région constante d'une immunoglobuline mammifère choisie à partir du groupe consistant en domaines humains d'IgE de C_{H}1, C_{H}2, C_{H}3 et C_{H}4, domaines d'IgM de C_{H}1, C_{H}2 et C_{H}3 et C_{H}4, domaines humains d'IgA de C_{H}1, C_{H}2 et C_{H}3, domaines humains d'IgG2 de C_{H}1, C_{H}2 et C_{H}3, domaines humains d'IgG3 de C_{H}1, C_{H}2 et C_{H}3, domaines humains d'IgG4 de C_{H}1, C_{H}2 et C_{H}3, domaines humains d'IgG1 de C_{H}1, C_{H}2 et C_{H}3, et domaines d'immunoglobuline constants dérivés à partir de souris, rats, chevaux, chiens, chats et d'autres mammifères, et un composant aviaire choisi à partir du groupe consistant en la partie extracellulaire d'un récepteur IgY Fc aviaire à haute affinité ou de la partie extracellulaire d'une molécule aviaire de la famille FcR, dans laquelle le peptide et le composant aviaire sont liés de manière covalente l'un à l'autre, et dans laquelle la construction est capable de détecter des anticorps IgY spécifiques d'antigène ou d'allergène en utilisant des procédés de détection pour des immunoglobulines mammifères.

2. Construction selon la revendication 1, dans laquelle au moins deux peptides différents compris dans la région constante d'une immunoglobuline mammifère sont prévus, dans laquelle les peptides différents de préférence sont compris dans des isotypes différents de l'immunoglobuline mammifère de la même espèce mammifère, ou sont compris dans des immunoglobulines différentes à partir d'espèces mammifères différentes.

3. Construction selon la revendication 1 ou 2, dans laquelle la construction est monomère ou homodimère.

4. Construction selon une des revendications 1 à 3, comprenant la partie extracellulaire du récepteur CHIR-AB1 des leucocytes aviaires.

5. Construction selon une des revendications 1 à 4, dans laquelle la construction est une protéine ou un peptide.

6. Acide nucléique isolé codant pour une construction selon une des revendications 1 à 5.

7. Vecteur d'expression comprenant comme séquence de codage un acide nucléique selon la revendication 6 et un promoteur, dans lequel l'acide nucléique est sous contrôle du promoteur.

8. Procédé de production d'une construction selon une des revendications 1 à 5, dans lequel une cellule, de préférence une cellule procaryotique ou eucaryotique, en particulier une cellule mammifère, est transformée avec le vecteur d'expression selon la revendication 6, dans lequel la cellule est cultivée pendant un temps prédéterminé et sous des conditions prédéterminées, et dans lequel pendant ou après la culture la construction est isolée du surnagé de la culture ou bien de la cellule.

9. Utilisation d'une construction selon une des revendications 1 à 5 pour la détection immunologique d'anticorps IgY.

10. Utilisation d'une construction selon une des revendications 1 à 5 pour la détection de réactifs de standardisation sur la base d'IgY dans des immunodosages pour la détermination d'anticorps mammifères.

11. Kit comprenant les composants suivants:
a) une construction selon une des revendications 1 à 5,
b) des anticorps IgY spécifiques d'antigène ou allergène,
c) optionnellement, des solutions de lavage et des solutions de tampon,
d) un réactif détecteur se liant à la construction,
dans lequel les composants a), b) et/ou d) peuvent être compris dans des paquets séparés ou dans un paquet unique.

12. Cellule hôte, en particulier cellule bactérienne, cellule de levure, cellule mammifère, cellule d'insecte, ou cellule de plante comprenant le vecteur d'expression selon la revendication 7.
